(19)

Europäisches
Patentamt
European
Patent Office
Office européen
des brevets

(11)     **EP 1 683 857 B1**

(12)     **EUROPEAN PATENT SPECIFICATION**

(45) Date of publication and mention
of the grant of the patent:
**16.02.2011   Bulletin 2011/07**

(21) Application number: **04792195.2**

(22) Date of filing: **08.10.2004**

(51) Int Cl.:
**C12N 5/07** (2010.01)

(86) International application number:
**PCT/JP2004/014926**

(87) International publication number:
**WO 2005/035737 (21.04.2005 Gazette 2005/16)**

(54) **METHOD OF PREPARING CELL CONCENTRATE AND CELL COMPOSITION**

VERFAHREN ZUR HERSTELLUNG EINES ZELLKONZENTRATS UND EINER
ZELLZUSAMMENSETZUNG

METHODE DE PREPARATION D'UN CONCENTRAT CELLULAIRE ET COMPOSITION
CELLULAIRE ASSOCIEE

(84) Designated Contracting States:
**AT BE BG CH CY CZ DE DK EE ES FI FR GB GR
HU IE IT LI LU MC NL PL PT RO SE SI SK TR**

(30) Priority: **10.10.2003   JP 2003352118
10.10.2003   JP 2003352729**

(43) Date of publication of application:
**26.07.2006   Bulletin 2006/30**

(73) Proprietor: **Asahi Kasei Kuraray Medical Co., Ltd.
Tokyo 101-8482 (JP)**

(72) Inventors:
• **TERASHIMA, Shuji
8700304 (JP)**

• **YASUTAKE, Mikitomo
8700165 (JP)**

(74) Representative: **von Kreisler Selting Werner
Deichmannhaus am Dom
Bahnhofsvorplatz 1
50667 Köln (DE)**

(56) References cited:
**EP-A- 1 300 168         WO-A-94/24555
WO-A-02/101029        WO-A1-98/32840
GB-A- 2 392 116         JP-A- 10 137 557
JP-A- 11 056 351         JP-A- 2000 166 541
JP-A- 2000 325 071     US-A1- 2003 134 416**

## Description

[TECHNICAL FIELD]

The present invention relates to: a method for preparing a cell concentrate, which comprises separating nucleated cells in a solution that contains cells, and concentrating them, so as to produce a cell suspension used for cryopreservation; a method for cryopreservation of cells; a cell composition; and a frozen cell product. More specifically, the present invention relates to: a method for preparing a cell concentrate, which is used to increase the concentration of nucleated cells in a cell suspension used for cryopreservation and to reduce the volume of the cell suspension used for cryopreservation; a method for cryopreservation of cells; a cell composition; and a frozen cell product

[BACKGROUND ART]

In recent years, transplantation of hematopoietic stem cells contained in the peripheral blood, bone marrow and cord blood, have vigorously been carried out for hematopoietic injury occurring as a side effect of chemotherapy for hematopoietic tumors such as leukemia and solid cancers. In almost all the cases, cells are preserved in a frozen state before transplantation. Since an enormous expense is required for such cryopreservation, components unnecessary for transplantation, such as erythrocytes or blood plasma, are generally eliminated before freezing, and thus a preservation volume is reduced. For example, approximately 100 ml of cord blood can be collected on average from the placenta and umbilical cord. In many cord blood banks, erythrocytes, blood plasma, and others are eliminated from such cord blood, so as to reduce the volume to 20 ml. Thereafter, 5 ml of a cryoprotective agent is added thereto, and thus the cord blood product is preserved in the volume of 25 ml. However, in many cases, such cord blood is preserved for a long period of time until the human leukocyte antigen (HLA) of a donor corresponds with that of a patient. Hence, in order to reduce an expense for preservation, needs for further reducing the volume of cord blood have grown.

Conventionally, as a method for separating components necessary for treatments from the blood, the specific gravity centrifugation, the erythrocyte agglutination method, the buffy coat method, the affinity separation method using an antibody, or the like have been applied. However, although the specific gravity centrifugation method has a high elimination rate of erythrocytes and blood plasma and has a high effect of reducing the volume, when a cell suspensions used as a raw material is laminated on a specific gravity solution (for example, Ficoll manufactured by Pharmacia), a solution surface should not be disturbed. Thus, this method involves complicated operations that require manipulative skills. Moreover, since the operations are carried out in an open system, this method has a high risk of contamination of various kinds of minor germs. Furthermore, this method is problematic in terms of a low recovery rate of cells of interest. In the erythrocyte agglutination method, erythrocytes are mixed with hydroxyethyl starch or the like, so that the erythrocytes can be agglutinated and precipitated due to the rouleau formation of erythrocytes, thereby separating a leukocyte layer as an upper layer (Proc. Natl. Acad. Sci. U.S.A., Vol. 92, pp. 10119-10122 (1995); Indian J. Med. Res., Vol. 106, pp. 16-19 (1997)). However, if the recovery rate of leukocytes is intended to increase, more erythrocytes are mixed and thus they interfere with reduction in the volume. In contrast, if the mixing of erythrocytes is intended to be suppressed, the recovery rate of leukocytes decreases. Thus, this method is problematic in that it cannot sufficiently satisfy the elimination of erythrocytes and the recovery of leukocytes at the same time. The buffy coat method comprises: strongly centrifuging a bag that stores the blood, so as to form an upper plasma layer, an intermediate buffy coat layer mainly comprising leukocytes and platelets, and a lower erythrocyte layer; and eliminating from the bag the upper plasma layer and the lower erythrocyte layer, so as to recover the intermediate buffy coat layer (Bone Marrow Transplantation, Vol. 23, pp. 505-509 (1999)). However, this method has the same problem as that of the erythrocyte agglutination method. The affinity separation method using an antibody has high specificity and a high effect of reducing the volume. However, since bounded antibody molecules should be subjected to an enzymatic treatment in order to recover the separated cells, this method is problematic in terms of damage to the cells, high expense, complicated operations, or the like.

As a simple cell separation method having relatively high cell separation efficiency, Japanese Patent Application Laid-Open (Kokai) Nos. 10-201470, 10-137557, 11-206875, and 11-56351 disclose a method using a filter device. In particular, Japanese Patent Application Laid-Open (Kokai) No. 11-56351 describes that after filtration of the blood, a rinsing solution is introduced into a filter device, so as to wash out erythrocytes remaining in the filter device, and that a recovery solution is then introduced into the filter device, so as to recover cells of interest captured in the filter device. Accordingly, this method brings on both a high elimination rate of erythrocytes and a high recovery rate of cells of interest. However, in the case of using this device, in order to maintain a high survival rate of the cells of interest or high functions thereof, addition of proteins such as albumin to the rinsing solution is necessary. Further, when such a rinsing solution is supplied, the air contained in blood tubes may enter into the filter device, resulting in incapacity for filtration (air block). Still further, in order not to enter the air into the filter device, it is necessary that the filter device and blood tubes have been primed with a rinsing solution before filtration. Thus, the use of the above filter device requires extremely complicated operations.

International Publication WO98/32840 describes that peripheral blood, bone marrow, cord blood, lymph, or a product produced by subjecting the above items to a certain treatment such as centrifugation, is used as a cell-containing solution. However, this publication does not describe at all that an efficiency of separating nucleated cells from unnecessary cells is increased by the treatment of a cell-containing solution before filtration.

Moreover, as a method for increasing a cell separation efficiency using a filter device, International Publication WO02/087660 discloses a method, which comprises: forming a blood cell concentration gradient in a unit of storing blood before filtration of the blood with a filter device; and filtrating the blood in the lower layer of the storage unit using a leukocyte-removing filter device. The technique disclosed in the above publication is as follows. A filter material is not sufficiently covered with a plasma protein suppressing the adhesion of blood cells, such as albumin, at an initial stage of filtration. Thus, large quantities of leukocytes to be eliminated adhere to the filter material, whereas platelets with a low specific gravity exist in the upper layer of a heterogenized whole blood preparation. When platelets are allowed to come into contact with the filter material, the surface of the filter material has been covered with a plasma protein such as albumin, and thus adhesion of platelets is suppressed. As a result, large quantities of platelets are recovered, and an efficiency of separating leukocytes from platelets is thereby increased. That is to say, differing from the present invention, the above publication does not include such a technical thought that a layer that is rich in unnecessary cells has previously been filtrated, that a part of nucleated cells are captured by the filter material, that unnecessary cells are run off, that nucleated cells are captured by the filter material, while unnecessary cells remaining on the filter material at that time are then washed out with a layer that is rich in nucleated cells, and then that a recovery solution is introduced into the filter device, so as to recover the nucleated cells.

As stated above, a filter method is an example of a simple cell separation method having relatively high cell separation efficiency. However, such a filter method has been problematic in that when the blood is filtrated, a filtration flow rate decreases as a result of the clogging of pores of a filter material due to fibrin clots or destroyed cell blood aggregates contained in the blood, so that the filtration time is prolonged, or in that a complete clogging results in incapability to conduct operations. As a mean against such problems, a method of expanding a filtration area, so as to reduce a filtration amount per unit cross section, is applied. As disclosed in the aforementioned filter method, in general, the blood is introduced into the filter device at a low flow rate during the filtration in order to prevent insufficiency in the capturing of cells of interest. On the other hand, a recovery solution is introduced into the filter device at a high flow rate during the recovery, in order to wash out the cells of interest captured by the filter material, utilizing the shearing force of the recovery solution. When a filtration area is expanded, if the recovery solution is introduced into the device at a high flow rate during the recovery, the recovery solution is not uniformly expanded to a filtration portion, but it preferentially flows around a recovery solution-introducing portion. Thus, the recovery rate of the cells of interest cannot be increased. That is to say, the filter method has been problematic in that it cannot simultaneously achieve the ensuring of a filtration flow rate and a high recovery rate of the cells of interest.

Among such filter methods, in the methods described in Japanese Patent Application Laid-Open (Kokai) Nos. 10-137557 and 11-206875, in order to increase the recovery rate of the cells of interest, the pore size of a porous body is greatened during recovery rather than during filtration, and the recovery solution is introduced into the filter device, so as to recover the cells of interest captured by the filter material. However, since the internal volume of the filter device increases during the recovery, in order to maintain a high recovery rate, the amount of the recovery solution to be introduced into the filter device must be practically increased. Thus, in order to obtain a desired volume, a centrifugal concentration becomes necessary. In addition, such methods have also been problematic in terms of a high expense for materials. Moreover, in such methods, the thickness of a filter material is controlled by compressing the filter material or releasing such compression, so as to change the pore size. However, such operations are complicated, and a precise control is difficult. Thus, such methods have also been problematic in terms of instability in the recovery rate of the cells of interest.

In almost all cases of transplantation of hematopoietic stem cells contained in peripheral blood, bone marrow, or cord blood, components necessary for treatments are separated from the blood by the aforementioned filter method or the like, and thereafter, the cells are preserved in a frozen state before transplantation. The separated cells are once placed in a container such as a blood bag, before they are transferred into a freezing container. Thereafter, the cells are subjected to various treatments such as washing, elimination of fluid components by centrifugation and concentration thereof, or addition of a cryoprotective agent. However, when the cells are transferred into another container, this operation has been problematic in terms of bacteria contamination or in that a cell concentrated solution is attached to the inside of the container and remain therein, resulting in the loss of cells. As a method for solving such problems, for the purpose of preventing the loss of cells or bacteria contamination caused by the transferring of the cells when the cultured cells are preserved in the frozen state, Japanese Patent Application Laid-Open (Kokai) No. 57-83284 discloses a technique of transferring the culture cells cultured in a culture bottle into a freezing container, centrifuging the cells therein, and then injecting a cryoprotective agent therein. However, since the technique disclosed in this publication is not carried out in a hermetically sealed system, it cannot sufficiently solve the problem regarding bacteria contamination.

Moreover, this technique does not comprise a step of discharging the air contained in the freezing container, and thus it cannot sufficiently reduce a cryopreservation volume due to the air remaining in the freezing container. Furthermore, this technique is also problematic in terms of the breakage of the freezing container occurring during thawing.

Further, the aforementioned International Publication WO98/32840 discloses a method of receiving the cells of interest separated by a filter device with a freezing bag, , so as to omit the operation to transfer the cells of interest. However, this publication does not describe at all that the cells of interest included in the freezing bag are centrifuged in that state, so as to concentrate nucleated cells.

As stated above, the existing techniques of separating the cells of interest from a cell suspension and concentrating them still have many problems to be solved. Thus, it is desired to be established a method for efficiently separating nucleated cells from unnecessary cells by simple operations, so as to reduce a cryopreservation volume.

[DISCLOSURE OF THE INVENTION]

[PROBLEMS TO BE SOLVED BY THE INVENTION]

It is an object of the present invention to provide: a method for preparing a cell concentrate which efficiently separates nucleated cells from a cell-containing solution that contains nucleated and unnecessary cells by simple operations, thereby reducing the volume of a solution used for cryopreservation that contains the nucleated cells, when the cell-containing solution that contains the nucleated cells and the unnecessary cells has been filtrated with a filter device and when a recovery solution is then introduced into the filter device to recover the nucleated cells captured by a filter material; and a cell composition. Moreover, it is another object of the present invention to provide: a method for preparing a cell concentrate which recovers nucleated cells at a high yield while suppressing a decrease in the filtration flow rate due to aggregates contained in the blood, and which can easily recover the nucleated cells of interest with a smaller amount of solution, when the cell-containing solution that contains nucleated cells and unnecessary cells has been filtrated with a filter device and when a recovery solution is then introduced into the filter device to recover the nucleated cells captured by a filter material; and a cell composition. Furthermore, it is a further object of the present invention to provide: a method for preserving cells in a frozen state, which can reduce the cryopreservation volume while reducing risks as the loss of nucleated cells, bacteria contamination, and the breakage of a freezing container, when nucleated cells are separated from a cell-containing solution that contains the nucleated cells and unnecessary cells, concentrated, and then cryopreserved; and a frozen cell product.

As a result of intensive studies directed towards achieving the aforementioned objects, the present inventors have found that in a method comprising filtrating a cell-containing solution that contains at least nucleated cells and unnecessary cells with a filter device, capturing the nucleated cells by a filter material, discharging the unnecessary cells from the filter device, and then introducing a recovery solution into the filter device, so as to recover the nucleated cells captured by the filter material, before filtration of the above cell-containing solution with the filter device, the above solution is separated into a layer that is rich in nucleated cells and a layer that is rich in unnecessary cells, and the layer rich in unnecessary cells and the layer rich in nucleated cells are filtrated in this order, so that the unnecessary cells remaining in the filter device can be eliminated from the filter device while the nucleated cells can be captured by the filter material, thereby simultaneously achieving the recovery of the nucleated cells at a high yield and elimination of the unnecessary cells at a high rate, and thereby reducing the above cell-containing solution to an extremely small volume by simple operations. Thus, the inventors have completed the present invention based on these findings.

Moreover, the present inventors have also found that in a method for separating and recovering the cells of interest, when a recovery solution-rectifying material is used together with a nucleated cell-capturing material such that the rectifying material has an arrangement that is specific for the capturing material, and when a relatively flat filter material having a specific ratio between an effective filtration membrane area and a filling thickness is used, two objects, namely, the maintenance of a flow rate during filtration and the recovery of nucleated cells at a high yield due to a uniform flow of the recovery solution, can be achieved, thereby completing the present invention.

Furthermore, the present inventors have also found that in a series of operations including separation of nucleated cells from a cell-containing solution that contains the nucleated cells and unnecessary cells, storage, centrifugal concentration, sealing, and cryopreservation, the steps except for separating the nucleated cells are carried out in a hermetically sealed single container, and at least the steps from storage up to cryopreservation are carried out in a hermetically sealed system, so as to reduce the risks as the loss of the nucleated cells and bacteria contamination. Further, the inventors have also found that the establishment of a hermetically sealed system that contains no air enables not only reduction in the cryopreservation volume, but also reduction of a risk of the breakage of a freezing container, thereby completing the present invention.

That is to say, the present invention provides the following features:

(1) A method for preparing a cell concentrate, which comprises: introducing a cell-containing solution that contains nucleated cells and unnecessary cells into a filter device comprising a filter material for substantially capturing the nucleated cells and for substantially giving passage to the unnecessary cells, so as to capture the nucleated cells by the above-described filter material and to discharge the unnecessary cells from the above device; and introducing a recovery solution into the above-described filter device, so as to recover the nucleated cells captured by the above-described filter material,

wherein the above-described method is characterized in that the cell-containing solution that contains nucleated cells and unnecessary cells are separated into a layer that is rich in nucleated cells and a layer that is rich in unnecessary cells, the layer rich in unnecessary cells is first introduced into the above-described filter device, and the layer rich in nucleated cells is then introduced therein, so as to discharge the unnecessary cells remaining in the above-described filter device while capturing the nucleated cells by the above-described filter material, and a recovery solution is then introduced into the above-described filter device, so as to recover the nucleated cells captured by the above-described filter material.

(2) The method for preparing a cell concentrate according to (1) above, wherein unnecessary cells are precipitated by gravity or centrifugal force, so as to separate a cell-containing solution that contains at least nucleated cells and unnecessary cells into a layer that is rich in nucleated cells and a layer that is rich in unnecessary cells.

(3) The method for preparing a cell concentrate according to (1) above, wherein unnecessary cells are agglutinated and are then precipitated by gravity or centrifugal force, so as to separate a cell-containing solution that contains at least nucleated cells and unnecessary cells into a layer that is rich in nucleated cells and a layer that is rich in unnecessary cells.

(4) The method for preparing a cell concentrate according to any one of (1) to (3) above, wherein the above-described unnecessary cells are erythrocytes.

(5) The method for preparing a cell concentrate according to any one of (1) to (4) above, wherein the above-described nucleated cells are hematopoietic stem cells.

(6) The method for preparing a cell concentrate according to (3) above, wherein unnecessary cells are agglutinated by adding hydroxyethyl starch to a cell-containing solution that contains nucleated cells and unnecessary cells.

(7) The method for preparing a cell concentrate according to any one of (1) to (6) above, wherein the above-described layer that is rich in nucleated cells consists of a nucleated cell-concentrated layer and a nucleated cell-diluted layer, and wherein the nucleated cell-diluted layer and the nucleated cell-concentrated layer are introduced into the filter device in this order.

(8) The method for preparing a cell concentrate according to any one of (1) to (5) above, wherein the above-described layer that is rich in nucleated cells consists of a nucleated cell-concentrated layer and a nucleated cell-diluted layer, and wherein the nucleated cell-concentrated layer and the nucleated cell-diluted layer are introduced into the filter device in this order.

(9) The method for preparing a cell concentrate according to (8) above, wherein a part of or all of the above-described nucleated cell-diluted layer is used as at least a portion of a recovery solution.

(10) The method for preparing a cell concentrate according to any one of (1) to (9) above, wherein a recovery solution is introduced into the above-described filter device to recover nucleated cells captured by the above-described filter material, and the recovered nucleated cell-containing solution is further centrifuged to eliminate the supernatant thereof, so as to concentrate the nucleated cells.

(11) The method for preparing a cell concentrate according to any one of (1) to (10) above, which is characterized in that the above-described filter material is configured such that a container having at least an inlet and an outlet for a cell-containing solution is packed with a nucleated cell-capturing material and a recovery solution-rectifying material, which consist of porous bodies, in this order in a direction from the inlet side to the outlet side for a cell containing solution, and in that the value obtained by dividing the effective filtration area of the above-described filter material by the thickness of the nucleated cell-capturing material packed is between 15 and 120 cm.

(12) A method for preparing a cell concentrate, which comprises: introducing a cell-containing solution that contains nucleated cells and unnecessary cells into a filter device comprising a filter material for substantially capturing the nucleated cells and for substantially giving passage to the unnecessary cells, so as to capture the nucleated cells by the above-described filter material and to discharge the unnecessary cells from the above device; and introducing a recovery solution into the above-described filter device, so as to recover the nucleated cells captured by the above-described filter material,

wherein the above-described method is characterized in that it uses a filter device formed by a container having an inlet and an outlet for a cell-containing solution with a filter material obtained by stacking a nucleated cell-capturing material and a recovery solution-rectifying material, which consist of porous bodies, wherein the value obtained by dividing the effective filtration area of the above-described filter material by the thickness of the nucleated cell-capturing material packed is between 15 and 120 cm, such that a nucleated cell-capturing material is located on the inlet side of a cell-containing solution, and in that the above method comprises introducing the cell-containing solution from the inlet therefor into the filter device, so as to capture nucleated cells by the filter material, discharging unnecessary cells from the above-described filter device, and introducing a recovery solution from the outlet side for a cell containing solution, so as to recover the nucleated cells captured by the above-described filter material from the inlet side for the cell containing solution.

(13) The method for preparing a cell concentrate according to (11) or (12) above, wherein an aggregate-capturing material is further packed to the cell-containing solution inlet side of the above-described nucleated cell-capturing material.

(14) The method for preparing a cell concentrate according to any one of (11) to (13) above, wherein the filter material is a non-woven fabric.

(15) The method for preparing a cell concentrate according to (14) above, which is characterized in that the nucleated cell-capturing material and the recovery solution-rectifying material, which consist of non-woven fabrics, are:

(i) a nucleated cell-capturing material consisting of a non-woven fabric having an average fiber diameter between 1.1 and 3.0 $\mu$m and a packing density between 0.1 and 0.3 g/cm$^3$; and
(ii) a recovery solution-rectifying material consisting of a non-woven fabric having an average fiber diameter between 0.5 and 1.5 $\mu$m and a packing density between 0.1 and 0.3 g/cm$^3$, and

which is characterized in that the average fiber diameter of the recovery solution-rectifying material is smaller than that of the nucleated cell-capturing material.

(16) The method for preparing a cell concentrate according to any one of (11) to (13) above, wherein the filter material is a sponge-like structure.

(17) The method for preparing a cell concentrate according to (16) above, which is characterized in that the nucleated cell-capturing material and the recovery solution-rectifying material, which consist of sponge-like structures, are:

(i) a nucleated cell-capturing material consisting of a sponge-like structure having an average pore diameter during packing between 7 and 25 $\mu$m and a porosity during packing between 55% and 90%; and
(ii) a recovery solution-rectifying material consisting of a sponge-like structure having an average pore diameter during packing between 2 and 10 $\mu$m and a porosity during filling between 55% and 90%, and

which is characterized in that the average pore diameter of the recovery solution-rectifying material is smaller than that of the nucleated cell-capturing material.

(18) The method for preparing a cell concentrate according to any one of (11) to (13) above, wherein the filter material is formed by the combination of a non-woven fabric with a sponge-like structure.

(19) A method for preserving cells in a frozen state, which further comprises the following steps, following the method for preparing a cell concentrate according to any one of (1) to (18) above:

(b) a cell storage step of transferring the recovered nucleated cells to a storage bag having a storage unit and a cryopreservation unit via a nucleated cell-introducing port established at the above-described storage unit, and storing them;
(c) a cell concentration step of centrifuging the nucleated cells stored in the above-described storage bag, while the cryopreservation unit is disposed at a position that is far from a rotation axis on a radius of gyration, so as to transfer the nucleated cells to the cryopreservation unit in the storage bag;
(d) a container separation step of hermetically sealing the concentrated nucleated cells in the cryopreservation unit in a state where it contains no air, so as to separate the cryopreservation unit by melting down; and
(e) a cryopreservation step of freezing the cryopreservation unit, in which the nucleated cells have been hermetically sealed, and preserving them,

wherein all of the above-described steps (b) to (e) are carried out in a hermetically sealed system.

(20) A method for preserving cells in a frozen state, which comprises at least the following steps (a) to (e):

(a) a step of preparing a cell concentrate, which comprises introducing a cell-containing solution that contains nucleated cells and unnecessary cells into a filter device comprising a filter material for substantially capturing the nucleated cells and for substantially giving passage to the unnecessary cells, so as to capture the nucleated cells by the above-described filter material and to discharge the unnecessary cells from the above device, and introducing a recovery solution into the above-described filter device, so as to recover the nucleated cells captured by the above-described filter material;
(b) a cell storage step of transferring the recovered nucleated cells to a storage bag having a storage unit and a cryopreservation unit via a nucleated cell-introducing port established at the above-described storage unit, and storing them;

(c) a cell concentration step of centrifuging the nucleated cells stored in the above-described storage bag, while the cryopreservation unit is disposed at a position that is far from a rotation axis on a radius of gyration, so as to transfer the nucleated cells to the cryopreservation unit in the storage bag;

(d) a container separation step of hermetically sealing the concentrated nucleated cells in the cryopreservation unit in a state where it contains no air, so as to separate the cryopreservation unit by melting down; and

(e) a cryopreservation step of freezing the cryopreservation unit, in which the nucleated cells have been hermetically sealed, and preserving them,

wherein the above-described steps (b) to (e) are carried out in a hermetically sealed system.

(21) The method for preserving cells in a frozen state according to (19) or (20) above, wherein the above-described unit for storing nucleated cells has a shape having an enlarged area, the cross section of which is gradually enlarged from the cryopreservation unit via a narrowed area.

(22) The method for preserving cells in a frozen state according to any one of (19) to (21) above, wherein, in the storage unit for storing the above-described nucleated cells, the narrowed area is used as a meltdown separation unit in the container separation step described in (d) above, and the cryopreservation unit is used as a container for cryopreservation of the nucleated cells in the cryopreservation step described in (e) above.

(23) The method for preserving cells in a frozen state according to any one of (19) to (22) above, wherein the cryopreservation unit of the above-described nucleated cells has a discharge port for the nucleated cells.

(24) The method for preserving cells in a frozen state according to any one of (19) to (23) above, wherein the cryopreservation unit of the above-described nucleated cells and/or the storage unit of the above-described nucleated cells have a cryoprotective agent-introducing unit.

(25) The method for preserving cells in a frozen state according to any one of (19) to (24) above, wherein the storage unit of the above-described nucleated cells has a filter device for discharging air contained in the bag for storing the nucleated cells.

(26) The method for preserving cells in a frozen state according to any one of (19) to (25) above, wherein the storage unit of the above-described nucleated cells has a conduit for discharging air contained in the bag for storing the nucleated cells, separately from a conduit for the nucleated cells.

(27) The method for preserving cells in a frozen state according to any one of (19) to (26) above, wherein a cryoprotective agent is added from the above-described cryoprotective agent-introducing unit before the container separation step described in (d) above.

(28) The method for preserving cells in a frozen state according to any one of (19) to (27) above, wherein a cryoprotective agent is added from the above-described cryoprotective agent-introducing unit after the container separation step described in (d) above.

(29) A method for producing a frozen cell product, which uses the method according to any one of (19) to (28) above.

(30) A cell composition, which is obtained by the method according to any one of (1) to (18) above.

(31) The cell composition according to (30) above, which is obtained by further centrifuging the above-described cell composition and eliminating a supernatant, so as to concentrate nucleated cells.

(32) The cell composition according to (30) or (31) above, wherein the above-described unnecessary cells are erythrocytes.

(33) The cell composition according to (30) or (31), wherein the above-described nucleated cells are hematopoietic stem cells.

(34) A frozen cell product, which is prepared by the method according to any one of (19) to (28).

[EFFECTS OF THE INVENTION]

Since the method for preparing a cell concentrate of the present invention enables efficient separation of nucleated cells from unnecessary cells contained in a cell-containing solution by simple operations, the cryopreservation volume can significantly be reduced. In addition, the present invention also enables the stable recovery of nucleated cells at a high yield from a cell-containing solution, while suppressing a decrease in the filtration flow rate of the cell-containing solution, and the easy recovery of the cells of interest with a smaller amount of solution. Moreover, in the present invention, since steps other than the step of separating nucleated cells are carried out in a hermetically sealed single container in a series of operations including separation of nucleated cells from a cell-containing solution that contains the nucleated cells and unnecessary cells, storage, centrifugal concentration, sealing, and cryopreservation, and since at least steps from storage up to cryopreservation are carried out in a hermetically sealed system, the cryopreservation volume can be reduced, while reducing the risks as the loss of the nucleated cells and the bacteria contamination. Furthermore, since the nucleated cells are hermetically sealed in a state where no air is contained, the present invention enables not only reduction in the cryopreservation volume, but also reduction of a risk of the breakage of a freezing container.

[BEST MODE FOR CARRYING OUT THE INVENTION]

Examples of a cell-containing solution that contains nucleated cells and unnecessary cells, which is used in the present invention, may include bone marrow, peripheral blood, peripheral blood mobilized by administration of a hematopoietic factor such as G-CSF, cord blood, products obtained by roughly separating these items by centrifugation or the like, and products obtained by diluting these items with a normal saline solution, a cell culture medium, a buffer solution, an anticoagulant or the like.

Examples of nucleated cells may include cells useful for cell therapy, diagnosis, etc., which are required for separation and recovery, such as leukocytes, mononuclear cells, lymphocytes, monocytes, macrophages, dendritic cells, granulocytes, hematopoietic stem cells, mesenchymal stem cells, vascular endothelial precursor cells, and nucleated erythrocytes.

On the other hand, examples of unnecessary cells may include cells, which cause adverse effects such as side effects on a recipient due to broken cells generated during a thawing process or the low recovery of cells to be recovered, if such cells exist with the cells to be recovered. Examples of such unnecessary cells may include erythrocytes, platelets, and granulocytes.

An example of the combination of nucleated cells with unnecessary cells in the present invention is a case where the nucleated cells are mononuclear cells and the unnecessary cells are erythrocytes. In this case, the mononuclear cells are captured by a filter material, whereas the erythrocytes pass through. Thereafter, the mononuclear cells captured by the filter material are recovered with a recovery solution.

The term "layer that is rich in nucleated cells" is used in the present invention to mean a layer that contains 50% or more, preferably 60% or more, more preferably 70% or more, and particularly preferably 80% or more of nucleated cells contained in a cell-containing solution before being separated into layers. If the ratio of such nucleated cells is less than 50%, the nucleated cells are captured by a filter material in the first half of filtration, and thereafter, the captured nucleated cells are continuously washed, thereby resulting in a decrease in the recovery rate of the nucleated cells. Thus, this is not favorable.

The term "layer that is rich in unnecessary cells" is used in the present invention to mean a layer that contains 60% or more, preferably 70% or more, and more preferably 80% or more of unnecessary cells contained in a cell-containing solution before being separated into layers. If the ratio of such unnecessary cells is less than 60%, large quantities of unnecessary cells are mixed in a layer that is rich in nucleated cells, which is to be filtrated later, and many unnecessary cells remain in a filter device. As a result, when nucleated cells captured by a filter material are recovered, many unnecessary cells are mixed in such cells, and it becomes difficult to reduce the volume of a nucleated cell suspension.

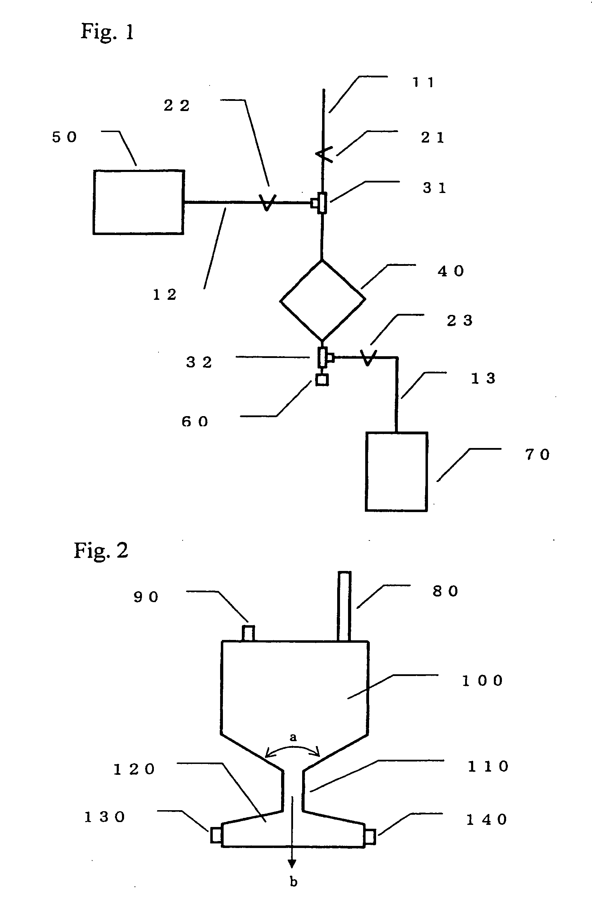

An example of the method for preparing a cell concentrate according to the present invention is a method using the system shown in Figure 1. Numbers 11, 12, and 13 indicate blood conduits, and numbers 21, 22, and 23 indicate clamps. Numbers 31 and 32 indicate T tubes. Number 40 indicates a cell separation filter device comprising a filter material (not shown in the figure) for substantially capturing nucleated cells and for substantially giving passage to unnecessary cells. Number 50 indicates a recovery bag for recovering the nucleated cells captured by the filter material of the cell separation filter device 40. Number 60 indicates a recovery solution-injecting port, and number 70 indicates a drain bag for storing unnecessary cells discharged from the cell separation filter device. First, the blood bag that stores a cell-containing solution is connected with the blood conduit 11, and the cell-containing solution is then separated into a layer containing nucleated cells and a layer containing unnecessary cells by a method such as centrifugation. Thereafter, the clamps 21 and 23 are opened. The layer rich in unnecessary cells is first introduced into the cell separation filter device 40, and the layer rich in nucleated cells is then introduced therein. In the present invention, nucleated cells can efficiently be separated from unnecessary cells. This is because the layer rich in unnecessary cells and the layer rich in nucleated cells are filtrated in this order, so that unnecessary cells, remaining in the cell separation filter device 40 can be discharged from the device, while nucleated cells are captured by the filter material of the cell separation filter device 40. When only the layer rich in nucleated cells is taken out after a cell-containing solution has been subjected to separation into layers and it is then filtrated by the cell separation filter device 40, for example, such operations are problematic in terms of the loss of cells due to transference and the mixing of unnecessary cells due to disturbance on the interface. Thus, these operations cannot achieve the recovery of nucleated cells at a high yield and elimination of unnecessary cells at a high rate at the same time. Moreover, when such a cell-containing solution is filtrated by the cell separation filter device 40 without separation into layers, many unnecessary cells remain in the above device, and thus the elimination rate of unnecessary cells cannot sufficiently be increased. That is to say, in order to increase the separation efficiency of cells, it is preferable that such a cell-containing solution be separated into a layer rich in nucleated cells and a layer rich in unnecessary cells, and that thereafter, the layer rich in unnecessary cells be introduced into the cell separation filter device 40, and the layer rich in nucleated cells be then introduced therein. The type of a method for introducing the layer rich in nucleated cells and the layer rich in unnecessary cells into the cell separation filter device 40 is not particularly limited. Examples of such a method may include dropping by head height gravity, pumping, and a method of squeezing the bag and then introducing the layers therein. During such introduction, if the layer rich in unnecessary cells is inclined towards the outlet side in the blood bag, the previously introduced unnecessary cells hardly remain therein. Thus, such a structure is preferable. The filtrated cell-containing solution is stored in the drain bag 7. After all the cell-containing solution contained in the blood bag has been introduced into the cell separation filter device 40, the clamps 21 and 23 are closed. Subsequently, the clamp 22 is opened. A recovery solution is introduced into the cell separation filter device 40 via a recovery solution-injecting port 60, and nucleated cells are then recovered into the recovery bag 50.

The type of the "method for separating a cell-containing solution that contains at least nucleated cells and the unnecessary cells into a layer that is rich in nucleated cells and a layer that is rich in unnecessary cells" is not particularly limited, and any method may be applied as long as it is a method capable of forming both layers in the unit for storing a cell-containing solution. As such a method, the free fall method utilizing gravity (which may be also referred to as a "static precipitation method") or centrifugation is relatively simple and thus preferable. Examples of the shape of the storage unit of a cell-containing solution may include a blood bag and a centrifuge tube. In terms of ease of introduction into a filter device, a blood bag is preferable.

In addition, it is more preferable that unnecessary cells be agglutinated before such separation into layers because it enables not only reduction in the time required for separation into layers but it also enables a further increase in the separation efficiency of nucleated cells from unnecessary cells. However, if the agglutinated unnecessary cells do not pass through a filter material, it results in a significant decrease in the separation efficiency of cells, and further it also causes a risk of provoking the clogging of the filter material. Accordingly, it is preferable that the size of the agglutinated unnecessary cells be smaller than the pore diameter of the filter material, or that such cells have a property of passing through the small pores of the filter material. When unnecessary cells are erythrocytes, a method of agglutinating erythrocytes with hydroxyethyl starch, dextran, or the like, can be applied. When such a polymer solution is attached to such an anionic erythrocyte surface, the repulsive force between blood cells is attenuated, and erythrocytes overlap with one another and become a state where rouleaux are connected with one another (which is called a rouleau formation), so as to promote precipitation. However, since the bond between erythrocytes is weak and thus such agglutination is easily decomposed by physical impact, the above method is preferably applied to a case where cells are filtrated after they have been agglutinated and precipitated, as in the present invention.

The terms "nucleated cell-concentrated layer" and "nucleated cell-diluted layer" are used in the present invention to mean that 70% or more, and preferably 80% or more of the nucleated cells contained in the layer rich in nucleated cells are contained in the nucleated cell-concentrated layer, and that the remaining nucleated cells are contained in the nucleated cell-diluted layer. When such nucleated cells are leukocytes and unnecessary cells are erythrocytes in the blood, for example, if the blood is centrifuged with strong centrifugal force of 3,000 G or greater, it is separated into the following three layers: an upper plasma layer (nucleated cell-diluted layer); an intermediate buffy coat layer that is rich in leukocytes (nucleated cell-concentrated layer); and a lower erythrocyte layer (that is rich in unnecessary cells) In this case, a part of or all of the upper plasma layer (nucleated cell-diluted layer) can be used as at least a portion of a recovery solution. In addition, when hydroxyethyl starch is added to the blood and the mixture is then left at rest, for example, it is separated into the following three layers: an upper leukocyte-concentrated layer (nucleated cell-concentrated layer); an intermediate leukocyte-diluted layer (nucleated cell-diluted layer); and a lower erythrocyte layer (that is rich in unnecessary cells). In this case, since the erythrocyte layer, the leukocyte-diluted layer, and the leukocyte-concentrated layer are introduced into a filter device in this order, a high concentration of leukocytes are introduced into the filter device in the latter half of filtration. Thereafter, more leukocytes are captured to the upstream of a filter material, and thus this means preferably creates a state where the recovery is most easily carried out. When the mean molecular weight of such hydroxyethyl starch is 400,000 and 10% normal saline solution is used, for example, the amount of hydroxyethyl starch added to the blood is preferably between 1/3 and 1/10 with respect to the blood as 1. The time for standing at rest is between 10 minutes and 80 minutes, and preferably between 20 minutes and 50 minutes. If the additive amount of hydroxyethyl starch is less than 1/10 and the time for standing at rest is shorter than 10 minutes, the rouleau formation of erythrocytes becomes insufficient, and separation into layers also becomes insufficient. Thus, this is unfavorable. On the other hand, if the additive amount of hydroxyethyl starch is more than 1/3 and the time for standing at rest is longer than 80 minutes, separation into layers rapidly progresses, and leukocytes become precipitated. In such a case, leukocytes are introduced into the filter device from the former half to the midpoint of filtration, and the leukocytes are likely to slide into the lower layer of the filter material. The recovery becomes difficult, and thus it is unfavorable.

The term "filter material" is used in the present invention to mean a porous filter material, which substantially captures nucleated cells and substantially gives passage to unnecessary cells. The expression "substantially capture nucleated cells" is used in the present invention to mean that the filter material captures 60% or more (more preferably 70% or more) of nucleated cells contained in a cell-containing solution. Moreover, the expression "substantially give passage to unnecessary cells" is used in the present invention to mean that 60% or more (more preferably 70% or more) of unnecessary cells contained in a cell-containing solution are allowed to pass through the filter material.

Any type of material is available for the filter material used in the present invention, as long as it is insoluble in water. In terms of excellent formability, excellent sterilization ability, and low cytotoxicity, preferred examples of such a material may include: synthetic polymers such as polyethylene, polypropylene, polystyrene, acrylic resin, nylon, polyester, polycarbonate, polyacrylamide, or polyurethane; natural polymers such as agarose, cellulose, cellulose acetate, chitin, chitosan, or alginic acid; inorganic materials such as hydroxyapatite, glass, alumina, or titania; and metals such as stainless, or titanium.

Examples of the shape of the filter material may include particles, a fibrous mass, a woven fabric, a non-woven fabric, a plate, and a sponge-like structure. Of these, a non-woven fabric and a sponge-like structure are preferable because of large surface area per volume.

When the filter material is a non-woven fabric, the average fiber diameter is between 1.0 and 30 $\mu$m, preferably between 1.0 and 20 $\mu$m, and more preferably between 1.5 and 10 $\mu$m. If the average fiber diameter is less than 1.0 $\mu$m, nucleated cells are too strongly attached to the filter material. As a result, it may make difficult to recover them, and it may also make difficult to give passage to unnecessary cells, especially when they are agglutinated. In contrast, if the average fiber diameter exceeds 30 $\mu$m, there is a high possibility that nucleated cells are not captured by fibers, but that they pass through. Both cases have a risk of leading to a decrease in the recovery rate, and thus they are not preferable.

In addition, when a sponge-like structure is used as a filter material, the average pore diameter is generally between 2.0 and 25 $\mu$m, preferably between 3.0 and 20 $\mu$m, and more preferably between 4.0 and 15 $\mu$m. If the average pore diameter is less than 2.0 $\mu$m, flow ability becomes deteriorated. It makes difficult to give passage to unnecessary cells, especially when they are agglutinated. Moreover, there is a risk that the supply of solutions becomes difficult. In contrast, if the average pore diameter exceeds 25 $\mu$m, the rate of capturing nucleated cells decreases, leading to a decrease in the recovery rate. Thus, this is not preferable.

The filter material used in the present invention is preferably a material produced by stacking a recovery solution-rectifying material on a nucleated cell-capturing material. The filter material, which are packed with the nucleated cell-capturing material and the recovery solution-rectifying material in this order in a direction from the inlet side towards the outlet side of a cell-containing solution in the filter device, enables the stable recovery of nucleated cells at a high yield from the cell-containing solution, while suppressing a decrease in the filtration flow rate of the cell-containing solution.

The term "nucleated cell-capturing material" is used in the present invention to mean a porous filter material having a function to capture nucleated cells contained in a cell-containing solution and to give passage to unnecessary cells. When such a nucleated cell-capturing material is a non-woven fabric, it is preferable that the average fiber diameter be between 1.1 and 3.0 $\mu$m, and that the packing density be between 0.1 and 0.3 g/cm$^3$. If the average fiber diameter is less than 1.1 $\mu$m and the packing density exceeds 0.3 g/cm$^3$, nucleated cells are captured only around the upstream of the nucleated cell-capturing material, and thus pores are occluded in a short time. As a result, the filtration flow rate decreases, and the peeling efficiency of nucleated cells also decreases, leading to a decrease in the recovery rate. Thus, this is not preferable. On the other hand, if the average fiber diameter is more than 3.0 $\mu$m and the packing density is less than 0.1 g/cm$^3$, nucleated cells cannot sufficiently be captured, and thus the cells reach the recovery solution-rectifying material disposed on the downstream side during filtration, so that they occlude pores. As a result, the filtration flow rate decreases, and the peeling efficiency of nucleated cells also decreases, leading to a decrease in the recovery rate. Thus, this is not preferable.

When the nucleated cell-capturing material has a sponge-like structure, it is preferable that the average pore diameter during packing be between 7 and 25 $\mu$m, and that the porosity during packing be between 55% and 90%. If the average pore diameter is less than 7 $\mu$m and the porosity during packing is lower than 55%, nucleated cells are captured only around the upstream of the nucleated cell-capturing material, and thus pores are occluded in a short time. As a result, the filtration flow rate decreases, and the peeling efficiency of nucleated cells also decreases, leading to a decrease in the recovery rate. Thus, this is not preferable. On the other hand, if the average pore diameter exceeds 25 $\mu$m and the porosity during packing exceeds 90%, nucleated cells cannot sufficiently be captured, and thus the cells reach the recovery solution-rectifying material disposed on the downstream side during filtration, so that they occlude pores. As a result, the flow rate of a cell-containing solution decreases during filtration, and the peeling efficiency of nucleated cells also decreases, leading to a decrease in the recovery rate. Thus, this is not preferable.

The term "recovery solution-rectifying material" is used in the present invention to mean a porous filter material having a function to uniformly spread a cell-containing solution to the filtration area of the filter device via filtration resistance, when a recovery solution is introduced into the filter device. When such a recovery solution-rectifying material is a non-woven fabric, it is preferable that the average fiber diameter be between 0.5 and 1.5 $\mu$m, and that the packing density be between 0.1 and 0.3 g/cm$^3$. If the average fiber diameter is less than 0.5 $\mu$m and the packing density exceeds 0.3 $\mu$g/cm$^3$, the filtration resistance increases, and the flow rate of the recovery solution decreases. As a result, the peeling rate of the captured nucleated cells decreases, and the recovery rate decrease. Thus, this is not preferable. On the other hand, if the average fiber diameter exceeds 1.5 $\mu$m and the packing density is less than 0.1 g/cm$^3$, the filtration resistance is small, and thus the recovery solution is not uniformly spread. As a result, the recovery rate of nucleated cells decreases. Thus, this is not preferable.

When such a recovery solution-rectifying material has a sponge-like structure, it is preferable that the average pore diameter during packing be between 2 and 10 $\mu$m, and that the porosity during packing be between 55% and 90%. If the average pore diameter is less than 2 $\mu$m and the porosity during packing is lower than 55%, the filtration resistance increases, and the flow rate of the recovery solution decreases. As a result, the peeling rate of the captured nucleated cells decreases, and the recovery rate decrease. Thus, this is not preferable. On the other hand, if the average pore diameter exceeds 10 $\mu$m and the porosity during packing exceeds 90%, the filtration resistance is small, and thus the recovery solution is not uniformly spread. As a result, the recovery rate of nucleated cells decreases. Thus, this is not preferable.

In the present invention, it is particularly important to use both the nucleated cell-capturing material and the recovery solution-rectifying material, which are disposed in a particular direction. It is necessary that the filter device be packed with the above nucleated cell-capturing material and recovery solution-rectifying material in this order in a direction from the inlet side of a cell-containing solution towards the outlet side thereof. By this structure, the flow of the recovery solution becomes uniform, and the recovery efficiency of the captured cells can be increased.

In addition, in the present invention, it is also preferable to add an aggregate-capturing material, as well as the aforementioned two types of materials, to the filter material. Such an aggregate-capturing material is packed to the side closest to the inlet for a cell-containing solution in the filter device, and can be used.

The term "aggregate-capturing material" is used in the present invention to mean a porous filter material, which is packed to the inlet side of a cell-containing solution of the nucleated cell-capturing material and which has functions to capture cell agglutinates such as fibrin clots, blood clots, activated platelets, or destroyed granulocytes in the cell-containing solution, and to suppress a decrease in the flow rate during filtration and a decrease in the nucleated cell recovery rate. When such a porous aggregate-capturing material is a non-woven fabric, it is preferable that the average fiber diameter be between 5 and 20 $\mu$m, and that the packing density be between 0.1 and 0.3 g/cm$^3$. If the average fiber diameter is less than 5 $\mu$m and the packing density is more than 0.3 g/cm$^3$, not only aggregates but also nucleated cells and unnecessary cells are captured, and pores are occluded with such cells. As a result, the filtration flow rate decreases, and nucleated cells are surrounded by aggregates, leading to a decrease in the recovery rate. Thus, this is not preferable. On the other hand, if average fiber diameter exceeds 20 $\mu$m and the packing density is less than 0.1 g/cm$^3$, aggregates cannot sufficiently be captured, and they reach the nucleated cell-capturing material disposed at the downstream during filtration and occlude the pores of the nucleated cell-capturing material. As a result, the filtration flow rate decreases, and nucleated cells are surrounded by aggregates, leading to a decrease in the recovery rate. Thus, this is not preferable.

When the aggregate-capturing material has a sponge-like structure, it is preferable that the average pore diameter during packing be between 60 and 150 $\mu$m, and that the porosity during packing be between 55% and 90%. If the average pore diameter is less than 60 $\mu$m and the porosity during packing be less than 55%, not only aggregates but also nucleated cells and unnecessary cells are captured, and pores are occluded with such cells. As a result, the flow rate of a cell-containing solution decreases during filtration, and nucleated cells are surrounded by aggregates, leading to a decrease in the recovery rate. Thus, this is not preferable. On the other hand, if the average pore diameter exceeds 150 $\mu$m and the porosity during packing exceeds 90%, aggregates cannot sufficiently be captured, and they reach the nucleated cell-capturing material disposed at the downstream during filtration and occlude the pores of the nucleated cell-capturing material. As a result, the filtration flow rate decreases, and nucleated cells are captured by aggregates, leading to a decrease in the recovery rate. Thus, this is not preferable.

The average fiber diameter is used in the present invention to mean a value obtained by the following procedures. That is to say, a part of a filter material, which constitutes such a filter material and is considered to be substantially uniform (for example, a nucleated cell-capturing material, a recovery solution-rectifying material, and an aggregate-capturing material) is sampled, and it is then photographed at a magnification between 1,000- and 3,000-folds using a scanning electron microscope or the like. For such sampling, the effective filtration cross section of the filter material is partitioned by squares with a side between 0.5 and 1 cm. Thereafter, 3 or more partitions, and preferably 5 or more partitions are then subjected to random sampling. For such random sampling, for example, addresses are assigned to the aforementioned partitions, and partitions more than necessary points may be selected by a method using the table of random numbers or the like. In addition, 3 or more points, and preferably 5 or more points are photographed in each of the sampled partitions. The diameters of all fibers seen in the thus obtained photographs are measured. The diameter is used herein to mean the width of a fiber that is in a direction at right angles to the fiber axis. The value obtained by dividing the sum of the measured diameters of all the fibers by the number of the fibers is defined as an average fiber diameter. However, when multiple fibers overlap with one another and the widths of fibers hidden behind other fibers cannot be measured, when multiple fibers form a thick fiber as a result of fusion, or further when fibers with significantly different diameters are mixed together, such data are deleted. By the above method, based on data from 500 or more fibers, and preferably from 1,000 or more fibers, an average fiber diameter is obtained.

The term "average pore diameter" is used in the present invention to mean that an analyte having a certain thickness obtained by cutting a portion at 0.5 mm or less to the thickness direction of a filter material from the surface of the filter material, as vertically as possible to the flow direction of the blood, is measured by the mercury penetration method (Shimadzu Corp., Pore Sizer 9320), and that when a state where no mercury is contained in the pores of the filter material is set at 0% of the amount of mercury penetrated, and when a state where mercury is contained in all the pores consisting of porous elements is set at 100% of the amount of mercury penetrated, a point corresponding to 50% of the amount of mercury penetrated is defined as the average pore diameter in the present invention. It is noted that measurement with a mercury porosimeter is carried out within the pressure range between 1 and 1,000 psia. Even in the case of a filter material, which is too flexible and thus when it is directly measured with a mercury porosimeter, it is deformed and pores cannot be detected, if the aforementioned measurement becomes possible as a result of preliminary adjustment such as immobilization for preventing the deformation of pores due to pressure, such a filter material is included in the present invention.

In the present invention, it is preferable to use a filter device to which a nucleated cell-capturing material and a recovery solution-rectifying material are packed in a specific direction. It is also preferable to use a filter material wherein the value obtained by dividing the effective filtration area thereof by the thickness of the packed nucleated cell-capturing material is between 15 and 120 cm. If such a value exceeds 120 cm, the effective filtration area becomes too wide with respect to the thickness of the nucleated cell-capturing material, and thus, a recovery solution is not uniformly spread to the filtration area during the recovery, leading to a decrease in the recovery rate of the nucleated cells. Thus, this is not preferable. On the other hand, if such a value is less than 15 cm, since the effective filtration area is narrow and the nucleated cell-capturing material is thick, it results in a decrease in the flow rate during filtration. In addition, since resistance becomes large when a recovery solution is introduced into the filter device, the flow rate of the recovery solution decreases, leading to a decrease in the recovery rate of the nucleated cells. Thus, this is not preferable.

Such a filter material may be directly used. However, as necessary, ligands exhibiting affinity for specific cells, such as amino acids, peptides, or glycoproteins (including bioligands such as antibodies or adhesion molecules) may be fixed thereon. Moreover, when platelets permeability is imparted to such a filter material, the surface of the filter material may be modified with synthetic polymers containing hydroxyethyl methacrylate as a main component or the like, as proposed in Japanese Patent Publication No. 6-51060.

The type of a container used for a filter device is not particularly limited. For example, a container having a shape such as a leukocyte-removing filter device as shown in Japanese Patent Publication No. 2-13588 can be used. Moreover, in such a container, it may also be possible to establish an inlet and an outlet for a recovery solution, in addition to the inlet and outlet for a cell-containing solution.

As a material for such a container, a material, which is insoluble in water and is excellent in formability and sterilization ability, and which has low cytotoxicity, is preferable. Moreover, a hard material, which does not substantially swell due to pressure charged into a filter device when a cell-containing solution is introduced into the filter device during filtration, or when a recovery solution is introduced therein during the recovery, is preferable used. If the container of the filter device swells during filtration, the flow rate of a recovery solution decreases, and shearing force for washing out nucleated cells captured by a filter material thereby decreases. In addition, a large amount of the recovery solution remains, causing a decrease in the recovery rate. Thus, this is not preferable. Moreover, there is also a risk of the breakage of the weld of a container. Examples of a preferred material may include polycarbonate, polyethylene, polypropylene, and polystyrene. However, preferred materials are not limited thereto, as long as they are hard and are suitable for medical purpose.

As a recovery solution used in the present invention, a solution, which hardly impairs nucleated cells and is capable of recovering such cells at a high yield, is preferable. Preferred examples of such a solution may include a commercially available normal saline solution, buffer solutions such as PBS (phosphate buffer solution) or HBSS (Hank's balanced salt solution), a cell culture medium such as RPMI1640, synthetic polymer solutions such as polyethylene glycol, polyvinylpyrrolidone, or polyvinyl alcohol, natural polymer solutions such as methylcellulose, gelatin, hydroxyethyl starch, dextran, a chitin derivative, collagen, fibronectin, albumin, or globulin, organic product solutions such as glucose, saccharose, maltose, sorbitol, glycerin, or dimethyl sulfoxide, and mixtures thereof. In addition, for the purpose of eliminating divalent cations to facilitate the peeling of cells, a chelating agent may be contained. Since a polymer solution such as dextran or hydroxyethyl starch also have functions as a cryoprotective agent, it can be cryopreserved directly or by addition of further cryoprotective agents such as DMSO. In addition, such a solution enables an increase in viscosity, when compared with a physiological saline or the like, and thus it improves shearing force during recovery. Thus, such a polymer solution gave good results in our evaluation, and it is more preferable as a recovery solution. Moreover, as described above, a part of or all of a nucleated cell-diluted layer obtained by separation of a cell-containing solution into layers can be used as at least a portion of such a recovery solution. In this case, since the recovery solution comprises autologous components, it does not damage to nucleated cells and there is no risk of virus infection. Thus, this is preferred.

A recovery method applied in the present invention is not particularly limited. In order to increase shearing force and to recover nucleated cells at a high yield, the flow rate of a recovery solution is preferably as high as possible. However, it is preferable that the flow rate be controlled to a rate, which does not cause disconnection of a connected portion between a filter device and a tube or the like due to an increase in the internal pressure or damage to nucleated cells. Moreover, examples of a means for introducing a recovery solution into a filter device may include: the use of devices such as a syringe pump, a blood pump, or a perista pump; a simple method of pushing a syringe by hand; a method of squeezing a bag that stores a liquid so as to provoke a flow; and a dropping by head height gravity. Furthermore, in order to further increase the recovery rate of nucleated cells, it may also be possible to give a vibration to a filter device, or to perform stopped-flow or the like. The direction for introducing a recovery solution into a filter device is the same direction as that in which a cell-containing solution is introduced, or a direction opposite thereto. In general, since the latter case brings on a higher recovery rate of cells, it is preferable.

In the present invention, in order to eliminate more unnecessary cells, the filter device may be rinsed after filtration. As a rinsing solution, a solution into which nucleated cells hardly leak and which washes out unnecessary cells, is preferable. Examples of such a rinsing solution may include a commercially available normal saline solution and buffer solutions such as D-PBS (Dalbecco's phosphate buffer solution) or HBSS (Hank's balanced salt solution). Such solutions may also comprise proteins such as albumin. In addition, the flow rate of such a rinsing solution is not particularly limited. In order to allow nucleated cells to stay on the upstream side of a filter material and to prevent such cells from incorporating into pores, it is preferable that the flow rate be as low as possible.

Moreover, a recovery solution is introduced into a filter device to recover nucleated cells, and the recovered nucleated cell-containing solution is then centrifuged to eliminate a supernatant, so that the nucleated cells can preferably be further concentrated.

The present invention further comprises a step of preserving in a frozen state a cell composition obtained by the aforementioned method for preparing a cell concentrate. In order to efficiently preserve in a frozen state a mononuclear cell fraction, which has been obtained at a high recovery rate by the aforementioned preparation method and which has a small volume of erythrocytes, it is preferable that the shape of a storage bag or a cryopreservation step be also devised. Such devices will be described below.

First, an example of the cell cryopreservation method of the present invention will be described in the case of cell separation using the system shown in Figure 1. In this example, number 50 of Figure 1 indicates a storage bag having a cryopreservation unit. As such a storage bag, a storage unit 100 having a cryopreservation unit 120 shown in Figure 2 is used.

First, clamps 21 and 22 are closed, and a cell-containing solution bag is connected with a blood conduit 11. Subsequently, the clamp 21 is opened, and the cell-containing solution is introduced into a cell separation filter device 40, which is packed with a filter material for capturing nucleated cells and giving passage to unnecessary cells, and the unnecessary cells are then discharged from the filter device. Thus, after all the cell-containing solution in the cell-containing solution bag has been introduced into the cell separation filter device 40 and the filtrated cell-containing solution has been discharged to a drain bag 70, the clamps 21 and 23 are closed. Subsequently, the clamp 22 is opened, and a recovery solution is introduced from a recovery solution-injecting port 60 into the cell separation filter device 40. Thus, while recovering the nucleated cells captured by the filter material, the cells are transferred into a storage bag 50 via a nucleated cell-introducing port 80 of the storage bag 50 connected with a blood conduit, and they are stored therein. When the nucleated cells are stored in the storage bag 50, the air contained in the storage bag 50 is discharged from an air-discharging filter device 90. Thereafter, the nucleated cell-introducing port 80 of the storage bag 50 is heat-sealed, and thus, the storage bag 50 is separated from the cell separation system by melting down. The thus separated storage bag 50 is fixed such that the cryopreservation unit 120 can be located at the bottom of a centrifuge cup, and it is then centrifuged. After the nucleated cells have been concentrated by centrifugation in the cryopreservation unit 120, a meltdown separation unit 110 is separated by a heat sealer, and the cryopreservation 120 is separated in a hermetically sealed state. Thereafter, a cryoprotective agent is introduced from a cryoprotective agent-introducing unit 130, and thereafter, the cryopreservation unit 120 is preserved in a frozen state.

With regard to the aforementioned cryopreservation method, in order to reduce a cryopreservation volume, it is preferable that the amount of unnecessary cells mixed in a cell suspension during cryopreservation be as small as possible. Specifically, the ratio of the volume of unnecessary cells to the volume of a nucleated cell-concentrated solution immediately before addition of a cryoprotective agent is preferably 65% or less, and more preferably 45% or less. If such a ratio exceeds 65%, when a supernatant is eliminated after centrifugal concentration of nucleated cells in a container separation step described later, nucleated cells approach to the liquid surface, or when a nucleated cell-concentrated solution is hermetically sealed, the above cells approach to the meltdown separation surface. Thus, it causes a high possibility of losing nucleated cells.

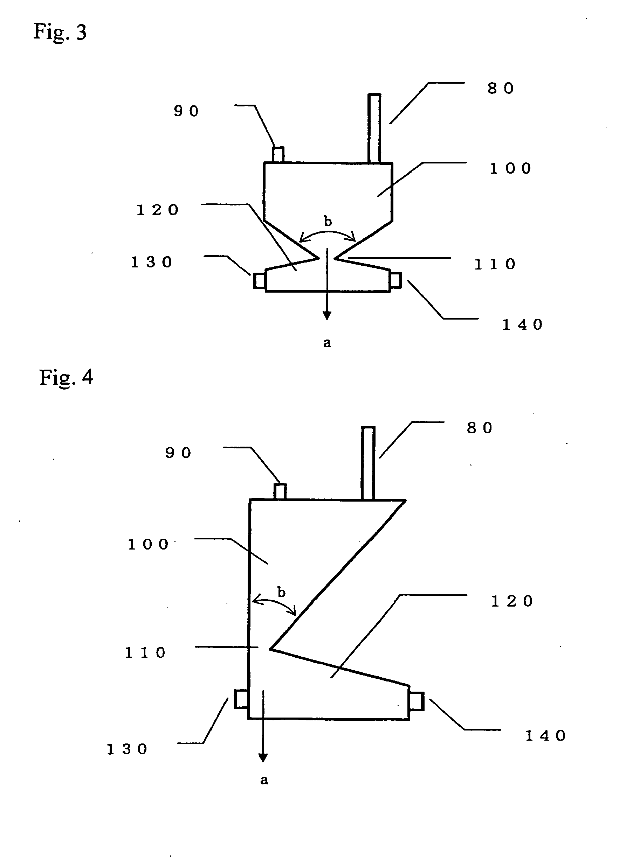

The "storage bag" used in the present invention comprises a cryopreservation unit, a storage unit, and a narrowed area for connecting them. A nucleated cell-introducing port is further attached to the above storage unit, and thus, the storage bag has a shape wherein nucleated cells can be moved through the narrowed area. Several examples of the shape of the storage bag are shown in Figures 2 to 4. In Figures 2 to 4, number 80 indicates a nucleated cell-introducing port, number 90 indicates an air-discharging filter device, number 100 indicates a storage unit, number 110 indicates a narrowed area (meltdown separation unit), number 120 indicates a cryopreservation unit, number 130 indicates a cryoprotective agent-introducing unit, and number 140 indicates a nucleated cell-discharging port.

The "storage unit" used in the present invention is a portion made from a flexible material, which is capable of storing nucleated cells. The nucleated cell-introducing port 80 may be attached to any position of the storage bag. From operational reasons, it is preferably attached to the storage unit 100.

The internal shape of the aforementioned storage bag is important. That is, as shown in the figures, if the storage bag has a shape having an enlarged area, the cross section of which is gradually enlarged from the cryopreservation unit 120 via the narrowed area (meltdown separation unit) 110 (a shape wherein a cross section is changed in a part of the storage unit 100), when centrifugal force is charged in the direction a, the cryopreservation unit 120 is easily separated by melting down at the narrowed area 110, and nucleated cells contained in the storage unit 100 are efficiently introduced into the cryopreservation unit 120. Thus, the above shape is preferable. Herein, the angle of the enlarged area (b in Figures 2 to 4), the cross section of which is gradually enlarged, is preferably between 20° and 150°, and more preferably between 45° and 120°. If such an angle is less than 20°, the storage unit 100 becomes long in a direction of the long side of a square, and as a result, handling deteriorates. For example, a storage bag cannot enter in a centrifuge cup during centrifugation. On the other hand, if such an angle is more than 150°, nucleated cells are accumulated in the enlarged area, and thus the cells cannot be sufficiently introduced into the cryopreservation unit 120, leading to the loss of cells. The storage unit 100 and the cryopreservation unit 120 may be three-dimensionally molded, such that they ensure space even in a state where the separated nucleated cell solution is not stored therein.

The "cryopreservation unit" used in the present invention is a portion having flexible properties with low temperature resistance, which stores nucleated cells separated from a cell-containing solution for a certain period of time, as with the storage unit, and which is then capable of storing nucleated cells concentrated by centrifugation. Examples of a material with low temperature resistance may include polyethylene, ethylene-vinyl acetate copolymer (EVA), fluorocarbon resins such as perfluoropropylene (FEP), and polyimide. However, examples are not limited thereto.

The "cell concentration step of centrifuging the nucleated cells stored in the storage bag, while the cryopreservation unit is disposed at a position that is far from a rotation axis on a radius of gyration" is used in the present invention to mean that the nucleated cells transferred into the storage bag are centrifuged, while the above storage bag that stores the above nucleated cells are fixed, such that strong centrifugal force can be loaded so as to concentrate the cells in the cryopreservation unit 120. Specifically, the cryopreservation unit 120 is oriented on the bottom side of a centrifuge cup during centrifugation, and it is fixed and then centrifuged.

The "container separation step of hermetically sealing the concentrated nucleated cells in the cryopreservation unit in a state where it contains no air, so as to separate the cryopreservation unit by melting down" is used in the present invention to mean that while discharging the air in the cryopreservation unit 120 to the storage unit 100 side, nucleated cells precipitated in the cryopreservation unit are sealed by giving a heat that is higher than the melting point of a freezing container material using a heat sealer, so as to prevent the leakage of the cells out of the cryopreservation unit, and that the cryopreservation unit 120 is then separated from the storage unit 100 using scissors, a cutter, or the like. An example of a method of discharging the air from the cryopreservation unit 120 is a method of turning up the narrowed area 110 for connecting the storage unit 100 with the cryopreservation unit 120 and holding with fingers a portion that contains the air or flicking fingers at such a portion, so as to discharge it to the storage unit 100 side. Moreover, if heat sealing is carried out at a pore portion without discharging a supernatant, the air hardly enters, and the movement of the liquid in the cryopreservation unit 120 is suppressed, so that nucleated cells are hardly lost. Thus, this means is preferable.

The "nucleated cell-discharging port" is used in the present invention to mean a low-temperature-resistant communicating unit, which is capable of communicating the inside of the cryopreservation unit 120 with the external space, and which has a function to discharge nucleated cells existing inside after the cryopreservation unit 120 has been subjected to a thawing treatment. Examples of such a discharging port may include a luer adapter capped with a screw cap, and a rubber stopper. However, examples are not limited thereto.

The "cryoprotective agent-introducing unit" is used in the present invention to mean a low-temperature-resistant communicating unit, which is capable of communicating the inside of the cryopreservation unit 120 with the external space, and which has a function to introduce a cryoprotective agent into the storage unit 100 or the cryopreservation unit 120. Examples of such an introducing unit may include a luer adapter capped with a screw cap, and a rubber stopper. However, examples are not limited thereto. In addition, in the case of this cryoprotective agent-introducing unit, after such a cryoprotective agent has been introduced into the storage unit 100 or the cryopreservation unit 120, if it is separated by meltdown separation using a heat sealer or the like, a projection can be removed from the cryopreservation unit 120. Thus, it is able to reduce a risk of breakage.

Examples of a cryoprotective agent may include dimethyl sulfoxide (DMSO), glycerin, dextran, hydroxyethyl starch (HES), and polyvinylpyrrolidone (PVP), but examples are not limited thereto. A right timing for introducing such a cryoprotective agent into the storage unit or cryopreservation unit is preferably before or after the container separation step because the amount of the agent introduced can be reduced. Since commonly used dimethyl sulfoxide has toxicity at ordinary temperature, if such a cryoprotective agent is introduced into the storage unit or cryopreservation unit before the cell concentration step, a contact time with the cryoprotective agent is prolonged, thereby giving damage to the cells. Thus, this is particularly not preferable.

The "air-discharging filter device" is used in the present invention to mean a portion, which is equipped in the storage unit 100 and discharges the air out of the storage unit 100. This filter device has a function to discharge the internal air to outside, as well as a function to prevent invasion of contaminants from outside. When the separated nucleated cells have been transferred into the storage unit 100 of a storage bag, not only the nucleated cell solution, but also the originally existing air and air entered when the nucleated cells have been transferred thereto, exist in the storage unit 100. Thus, there is a risk that the storage unit 100 and the cryopreservation unit 120 expand and burst. Accordingly, by equipping the storage unit 100 with the air-discharging filter device, such bursting can preferably be prevented. A porous membrane having action to eliminate bacteria used as a filter material is preferably able to prevent the influx of bacteria into the storage unit 100. This porous membrane has a pore diameter, which facilitates aeration and is able to prevent the influx of bacteria or viruses. A pore diameter is preferably between 0.1 and 0.6 $\mu$m. Moreover, it is preferable that such a porous membrane does not get wet even if it comes into contact with a cell-containing solution and be of a hydrophobic material capable of aeration. Furthermore, for the same purpose, an "air-discharging conduit," as well as a nucleated cell conduit, may be established in the storage unit 100. This conduit is a closed system and is capable of connecting the air in the storage unit 100 with a blood bag or a system used in the cell separation step, so as to discharge it.

The "cryopreservation step of freezing the cryopreservation unit, in which the nucleated cells have been hermetically sealed, and preserving them" in the present invention is not particularly limited, as long as it is a method that is commonly used as a cryopreservation method of cells. In order to prevent breakage due to impact such as falling, it is preferable that the cryopreservation unit 120 be protected by being placed in a metal canister. In order to reduce damage to cells, it is preferable that the cryopreservation unit 120 be cryopreserved in a program freezer before preservation with liquid nitrogen. Cryopreservation with liquid nitrogen may be carried out either in a gas phase or in a liquid phase.

In the cell cryopreservation method of the present invention, it is preferable that a treatment up to cryopreservation of nucleated cells, after the nucleated cells have been separated from a cell suspension, be carried out in a hermetically sealed system in a single container. The "hermetically sealed system" in the present invention can adopt any structure, as long as it is a system closed from the outside world, so as to reduce the loss of nucleated cells and a risk of bacteria contamination. Such a hermetically sealed system can preferably adopt a structure wherein nucleated cells are not allowed to come into contact with the outside air during the steps (b) to (e). More preferably, if a cell separation system used in the filter method is connected with a cryopreservation container, all the steps ranging from cell separation to cryopreservation can be carried out in a hermetically sealed system, and as a result, a risk of contamination is significantly reduced.

The present invention will be more specifically described in the following examples. However, these examples are not intended to limit the scope of the present invention.

[EXAMPLES]

Methods of separating mononuclear cells from cord blood, concentrating them, and adjusting the final volume thereof to 4 cm$^3$, will be described in the following Examples 1 and 2 and Comparative examples 1 to 4. In these cases, unnecessary cells are erythrocytes.

[Example 1] Method of filtrating layers separated from cord blood using an erythrocyte agglutination reagent

(1) Production of cell separation filter device

The following container was prepared: a polycarbonate container consisting of an upper container and a lower container, which has an inside dimension after fabrication consisting of 43 mm long, 43 mm wide, and 2.9 mm high (effective filtration area: 18.5 cm$^2$; internal volume: 7 cm$^3$), and which has a liquid outlet and a liquid inlet on the longest diagonal line. A filter material was produced from: 0.14 g of a polyester non-woven fabric with an average fiber diameter of 12 $\mu$m acting as a first aggregate-capturing material; 1.28 g of a polyester non-woven fabric with an average fiber diameter of 1.7 $\mu$m acting as a second nucleated cell-capturing material; and 0.19 g of a polyester non-woven fabric with an average fiber diameter of 1.1 $\mu$m acting as a third recovery solution-rectifying material.

Thereafter, from the inlet side of the above polycarbonate container, the above filter material was packed into the container. It is to be noted that the packing densities of the first, second, and third layers were 0.20 g/cm$^3$, 0.24 g/cm$^3$, and 0.20 g/cm$^3$, respectively. For the purpose of imparting platelets permeability to the above filter material, the material was coated with a hydrophilic polymer. That is to say, an ethanol solution that contained 1 % of a hydroxyethyl methacrylate-dimethylaminoethyl methacrylate copolymer (97 : 3 at a molar ratio) was supplied from the liquid inlet of the above cell separation filter device, and an extra polymer solution was then purged with nitrogen gas. Thereafter, it was dried with a vacuum dryer at 60°C for 8 or more hours.

(2) Cell separation system

The cell separation system shown in Figure 1 was used.

(3) Cell concentration operation

Clamps 21 and 22 had previously been closed. 20 cm$^3$ of 6% hydroxyethyl starch (Hishiyama Seiyaku, "HES40 (erythrocyte-precipitating agent)") was added to 100 cm$^3$ of CPD-added human cord blood (hematocrit value: 27.8%) stored in a 200-cm$^3$ blood bag, and they were then mixed for 10 minutes. Thereafter, the blood bag was connected with a blood conduit 11, and it was then suspended, followed by hanging it at rest for 1 hour. After confirming that erythrocytes had been precipitated in the blood bag and that the interface between the upper leukocyte layer and the lower erythrocyte layer had become clear, the clamp 21 was opened, and filtration was initiated by head height gravity. This time, it was observed that the lower erythrocyte layer was first flown to the cell separation filter device 40, that the upper leukocyte layer was then flown thereto, and that erythrocytes remaining in the cell separation filter device 40 were washed out. The filtrated blood was stored in a drain bag 70. After confirming that all the blood in the blood bag had been introduced into the cell separation filter device 40, the clamps 21 and 23 were closed. Subsequently, human serum albumin was added to a 10% dextran normal saline solution (Kobayashi Pharmaceutical Co., Ltd., "Dextran 40 Injection") to a final concentration of 3%. A syringe was then filled with 19 cm$^3$ of the obtained solution and 18 cm$^3$ of air. Thereafter, the clamp 22 was opened, the solution and then the air were manually supplied from a recovery solution-injecting port 60, and mononuclear cells were recovered in a recovery bag 50 (recovered mononuclear cell solution 1). In this case, the amount of a cell solution recovered was 23 cm$^3$ the hematocrit value was 3.5%, and the time required for recovery was 2 seconds (flow rate: 570 cm$^3$/min). Thereafter, the recovered mononuclear cells were transferred into a conical tube, and they were then centrifuged under centrifugal conditions of 400 G x 15 minutes. The obtained supernatant was eliminated, resulting in a final volume of 4 cm$^3$, and the precipitate was dispersed by shaking it upside down (recovered mononuclear cell solution 2).

(4) Analysis

In the present cell concentration operation, the number of cells was counted using a sequential multi channel automatic blood cell analyzer (SF3000, Sysmex). The volume of erythrocytes in the recovered mononuclear cell solution 1, the recovery rate 1 of mononuclear cells, and the recovery rate 2 of mononuclear cells were calculated using the following formulas:

$$\text{Erythrocyte volume (cm}^3) = (\text{hematocrit value of recovered mononuclear cell solution 1} \times \text{liquid amount of recovered mononuclear cell solution 1}) / 100$$

$$\text{Mononuclear cell recovery rate 1 (\%)} = 100 \times (\text{number of mononuclear cells in recovered mononuclear cell solution 1} / \text{number of mononuclear cells in cord blood})$$

$$\text{Mononuclear cell recovery rate 2 (\%)} = 100 \times (\text{number of mononuclear cells in recovered mononuclear cell solution 2} / \text{number of mononuclear cells in cord blood})$$

(5) Results

In the present cell concentration operation, the volume of erythrocytes was 0.8 cm$^3$, the recovery rate 1 of mononuclear cells was 85%, and the recovery rate 2 of mononuclear cells was 82%.

[Example 2] Method of filtrating layers separated from cord blood using strong centrifugal force

(1) Production of cell separation filter device

The same device as that used in Example 1 was used.

(2) Cell separation system

The cell separation system shown in Figure 1 was used.

(3) Cell separation operation

Clamps 21, 22 and 23 of the cell separation system shown in Figure 1 had previously been closed. A 200-$cm^3$ blood bag that stored 100 $cm^3$ of CPD-added human cord blood (hematocrit value: 31.1%) was connected with a blood conduit 11, and the blood bag, which was in a state where it stood vertically, was then fixed to a centrifuge cup. The blood bag was centrifuged at 3,000 G for 12 minutes at 10°C. Thereafter, the blood bag and the cell separation system were gently taken out from the centrifuge cup, and the blood bag was then hanged. At this time, the contents in the blood bag were separated into the following 3 layers: an upper plasma layer; an intermediate buffy coat layer; and a lower erythrocyte layer. 19 $cm^3$ of the upper plasma layer, which was to be used as a recovery solution, was collected into a syringe, without disturbing the interface. Thereafter, the clamps 21 and 23 were opened, and filtration was initiated by head height gravity. This time, it was observed that the lower erythrocyte layer was first flown to the cell separation filter device 40, that the intermediate buffy coat layer, and then the upper plasma layer were successively flown thereto, and that erythrocytes remaining in the cell separation filter device 40 were washed out. The filtrated blood was stored in a drain bag 70. After confirming that all the blood in the blood bag had been introduced into the cell separation filter device 40, the clamps 21 and 23 were closed. Subsequently, 18 $cm^3$ of air was added to the plasma, which had previously been collected in a syringe, and the clamp 22 was then opened. A liquid and then an air were manually supplied from a recovery solution-injecting port 60, and mononuclear cells were recovered in a recovery bag 50 (recovered mononuclear cell solution 1). In this case, the amount of a cell solution recovered was 23 $cm^3$, the hematocrit value was 0.12%, and the time required for recovery was 2 seconds (flow rate: 570 $cm^3$/min). Thereafter, the recovered mononuclear cells were transferred into a conical tube, and they were then centrifuged under centrifugal conditions of 400 G x 15 minutes. The obtained supernatant was eliminated, resulting in a final volume of 4 $cm^3$, and the precipitate was dispersed by shaking it upside down (recovered mononuclear cell solution 2).

(4) Results

In the present cell concentration operation, the volume of erythrocytes was 0.5 $cm^3$, the recovery rate 1 of mononuclear cells was 87%, and the recovery rate 2 of mononuclear cells was 84%.

[Comparative example 1] Method of filtrating cord blood without separating it into layers

(1) Production of cell separation filter device

The same device as that used in Example 1 was used.

(2) Cell separation system

The cell separation system shown in Figure 1 was used.

(3) Cell separation operation

100 $cm^3$ of cord blood (hematocrit value: 28.2%) was directly introduced into the cell separation filter device 40, without separating it into layers. Thereafter, the same operations as those conducted in Example 1 were conducted. The amount of a cell solution recovered was 23 $cm^3$, the hematocrit value was 9.1 %, and the time required for recovery was 2 seconds (flow rate: 570 $cm^3$/min).

(4) Results

In the present cell concentration operation, the volume of erythrocytes was 2.1 $cm^3$ the recovery rate 1 of mononuclear cells was 85%, and the recovery rate 2 of mononuclear cells was 60%.

[Comparative example 2] Method 1 of separating mononuclear cells from cord blood by erythrocyte agglutination method

20 $cm^3$ of 6% hydroxyethyl starch (Hishiyama Seiyaku, "HES40 (erythrocyte-precipitating agent)") was added to 100 $cm^3$ of CPD-added human cord blood (hematocrit value: 29.6%) stored in a 200-$cm^3$ blood bag, and they were then mixed for 10 minutes. Thereafter, the blood bag was fixed to a centrifuge cup such that it could stand vertically, and it was then centrifuged at 50 G x 5 minutes at 10°C. Subsequently, the blood bag was pressed with a separation stand, so as to suck up as less as possible erythrocyte layer, and 60 cm$^3$ of the upper leukocyte layer was collected (recovered mononuclear cell solution 1). The obtained layer was then transferred into a conical tube. The hematocrit value of the recovered mononuclear cell solution 1 was 2.5%. The mononuclear cells were then centrifuged at 400 G x 10 minutes at 10°C. Thereafter, the obtained supernatant was eliminated, and 4 ml of the remaining precipitate was dispersed by shaking it upside down (recovered mononuclear cell solution 2). In the present cell concentration operation, the volume of erythrocytes was 1.5 cm$^3$, the recovery rate 1 of mononuclear cells was 66%, and the recovery rate 2 of mononuclear cells was 58%.

[Comparative example 3] Method 2 of separating mononuclear cells from cord blood by erythrocyte agglutination method

20 cm$^3$ of 6% hydroxyethyl starch (Hishiyama Seiyaku, "HES40 (erythrocyte-precipitating agent)") was added to 100 cm$^3$ of CPD-added human cord blood (hematocrit value: 33.3%) stored in a 200-cm$^3$ blood bag, and they were then mixed for 10 minutes. Thereafter, the blood bag was fixed to a centrifuge cup such that it could stand vertically, and it was then centrifuged at 50 G x 5 minutes at 10°C. Subsequently, the blood bag was pressed with a separation stand, so as to collect 65 cm$^3$ of an upper leukocyte layer including a lower erythrocyte layer (recovered mononuclear cell solution 1). The thus recovered solution was then transferred into a conical tube. The hematocrit value of the recovered mononuclear cell solution 1 was 10%. The mononuclear cells were then centrifuged at 400 G x 10 minutes at 10°C. Thereafter, the obtained supernatant was eliminated, and 4 ml of a precipitate was obtained (recovered mononuclear cell solution 2). In the present cell concentration operation, the volume of erythrocytes was 6.5 cm$^3$, the recovery rate 1 of mononuclear cells was 85%, and the recovery rate 2 of mononuclear cells was 32%.

[Comparative example 4] Method of separating mononuclear cells by separation of cord blood into layers with strong centrifugal force

100 cm$^3$ of CPD-added human cord blood (hematocrit value: 27.3%) was stored in a 200-cm$^3$ blood bag having discharging conduits at upper and lower portions, and the blood bag was then fixed to a centrifuge cup such that it could stand vertically, followed by centrifugation at 3,000 G x 12 minutes at 10°C. Subsequently, the blood bag was pressed with a separation stand, so as to discharge an upper plasma layer from the top of the blood bag and discharge a lower erythrocyte layer from the bottom thereof. As a result, 30 cm$^3$ of the blood including a buffy coat layer remained in the blood bag (recovered mononuclear cell solution 1). The hematocrit value of the recovered mononuclear cell solution 1 was 40%. The recovered mononuclear cell solution 1 was transferred into a conical tube, and was then centrifuged at 400 G x 10 minutes at 10°C. Thereafter, the obtained supernatant was eliminated, and 4 ml of a precipitate was obtained (recovered mononuclear cell solution 2). In the present cell concentration operation, the volume of erythrocytes was 12.0 cm$^3$, the recovery rate 1 of mononuclear cells was 95%, and the recovery rate 2 of mononuclear cells was 21%.
The results of the above examples and comparative examples are summarized in Table 1. As is clear from the results shown in Table 1, the methods described in the examples of the present invention enable reduction in the volume of erythrocytes as well as the achievement of a high mononuclear cell recovery rate.

[Table 1]

| | Volume of erythrocytes | Mononuclear cell recovery rate 1 | Mononuclear cell recovery rate 2 |
|---|---|---|---|
| Example 1 | 0.8 cm$^3$ | 85% | 82% |
| Example 2 | 0.5 cm$^3$ | 87% | 84% |
| Comparative example 1 | 2.1 cm$^3$ | 85% | 60% |
| Comparative example 2 | 1.5 cm$^3$ | 66% | 58% |
| Comparative example 3 | 6.5 cm$^3$ | 85% | 32% |
| Comparative example 4 | 12.0 cm$^3$ | 95% | 21 % |

Hereafter, methods of separating mononuclear cells from cord blood and concentrating them will be described in the following Examples 3 to 6 and Comparative examples 5 to 9. In these cases, unnecessary cells are erythrocytes.

[Example 3]

(1) Production of cell separation filter device

The following container was prepared: a polycarbonate container consisting of an upper container and a lower container, which has an inside dimension after fabrication consisting of 43 mm long, 43 mm wide, and 2.9 mm high (effective filtration area: 18.5 cm$^2$; internal volume: 7 cm$^3$), and which has a liquid outlet and a liquid inlet on the longest diagonal line. A filter material was produced from: 1.37 g of a polyester non-woven fabric with an average fiber diameter of 1.7 $\mu$m acting as a first nucleated cell-capturing material; and 0.19 g of a polyester non-woven fabric with an average fiber diameter of 1.1 $\mu$m acting as a second recovery solution-rectifying material. Thereafter, from the inlet side of the above polycarbonate container, the above filter material was packed into the container such that it was packed around the upper container and around the lower container, so as to separate the space on the container inlet side from the space on the container outlet side. It is to be noted that the packing thickness and packing density of the first layer were 2.5 mm and 0.24 g/cm$^3$, and that the packing thickness and packing density of the second layer were 0.4 mm and 0.20 g/cm$^3$, respectively. The value obtained by dividing the effective filtration area by the thickness of the nucleated cell-capturing material packed was 74 cm. In addition, for the purpose of imparting platelets permeability to the above filter material, the material was coated with a hydrophilic polymer. That is to say, an ethanol solution that contained 1% of a hydroxyethyl methacrylate-dimethylaminoethyl methacrylate copolymer (97 : 3 at a molar ratio) was supplied from the liquid inlet of the above cell separation filter device, and an extra polymer solution was then purged with nitrogen gas. Thereafter, it was dried with a vacuum dryer at 60°C for 8 or more hours.

(2) Cell separation system

The cell separation system shown in Figure 1 was used.

(3) Cell separation operation

100 cm$^3$ of CPD-added human cord blood was supplied by head height gravity to the cell separation filter device from the inlet for a cell-containing solution, so as to capture mononuclear cells. The filtrated blood was recovered in a drain bottle. Subsequently, human serum albumin was added to a commercially available 10% dextran normal saline solution (Kobayashi Pharmaceutical Co., Ltd., "Dextran 40 Injection") to a final concentration of 3%. A syringe was then filled with 19 cm$^3$ of the thus obtained solution and 18 cm$^3$ of air. Thereafter, the solution was manually supplied from the outlet for a cell-containing solution, so as to recover mononuclear cells from the outlet for the cell-containing solution. In this case, the amount of a cell solution recovered was 23 cm$^3$, and the time required for recovery was 2 seconds (flow rate: 570 cm$^3$/min).

(4) Analysis

In the present cell concentration operation, the number of cells was counted using a sequential multi channel automatic blood cell analyzer (SF3000, Sysmex). The mononuclear cell recovery rate was calculated using the following formula: Mononuclear cell recovery rate (%) = 100 x (number of mononuclear cells in recovered cell solution / number of mononuclear cells in cord blood)

(5) Results

In the present cell separation operation, the mononuclear cell recovery rate was 85%, and the filtration time was 6 minutes.

[Example 4]

(1) Production of cell separation filter device

The following container was prepared: a polycarbonate container consisting of an upper container and a lower container, which has an inside dimension after fabrication consisting of 30 mm long, 30 mm wide, and 12.4 mm high (effective filtration area: 9 cm$^2$; internal volume: 11 cm$^3$), and which has a liquid outlet and a liquid inlet on the longest diagonal line. A filter material was produced from: 1.32 g of a polyester non-woven fabric with an average fiber diameter of 2.3 $\mu$m acting as a first nucleated cell-capturing material; and 0.19 g of a polyester non-woven fabric with an average fiber diameter of 1.1 $\mu$m acting as a second recovery solution-rectifying material. Thereafter, from the inlet side of the above polycarbonate container, the above filter material was packed into the container such that it was packed around the upper container and around the lower container, so as to separate the space on the container inlet side from the space on the container outlet side. It is to be noted that the packing thickness and packing density of the first layer were 5.4 mm and 0.20 g/cm$^3$, and that the packing thickness and packing density of the second layer were 7 mm and 0.20 g/cm$^3$, respectively. The value obtained by dividing the effective filtration area by the thickness of the nucleated cell-capturing material filled was 16.7 cm. In addition, as in the case of Example 1, the non-woven fabric was coated with a hydrophilic polymer.

(2) Cell separation system

The cell separation system shown in Figure 1 was used.

(3) Cell separation operation

The same cell separation operation as that in Example 3 was carried out. The amount of a cell solution recovered was 21 cm$^3$, and the time required for recovery was 3 seconds (flow rate: 380 cm$^3$/min).

(4) Results

In the present cell separation operation, the mononuclear cell recovery rate was 75%, and the filtration time was 10 minutes (flow rate: 10 cm$^3$/min).

[Example 5]

(1) Production of cell separation filter device

The same container as used in Example 3 was used. A filter material was produced from: 0.14 g of a polyester non-woven fabric with an average fiber diameter of 12 $\mu$m acting as a first aggregate-capturing material; 1.28 g of a polyester non-woven fabric with an average fiber diameter of 1.7 $\mu$m acting as a second nucleated cell-capturing material; and 0.19 g of a polyester non-woven fabric with an average fiber diameter of 1.1 $\mu$m acting as a third recovery solution-rectifying material. Thereafter, from the inlet side of the above container, the above filter material was packed . It is to be noted that the packing thickness and packing density of the first layer were 0.3 mm and 0.20 g/cm$^3$, that the packing thickness and packing density of the second layer were 2.2 mm and 0.24 g/cm$^3$ and that the packing thickness and packing density of the third layer were 0.4 mm and 0.20 g/cm$^3$, respectively. The value obtained by dividing the effective filtration area by the thickness of the nucleated cell-capturing material packed was 84.1 cm. In addition, as in the case of Example 3, the non-woven fabric was coated with a hydrophilic polymer.

(2) Cell separation system

The cell separation system shown in Figure 1 was used.

(3) Cell separation operation

The same cell separation operation as that in Example 3 was carried out. The amount of a cell solution recovered was 23 cm$^3$, and the time required for recovery was 2 seconds (flow rate: 570 cm$^3$/min).

(4) Results

In the present cell separation operation, the mononuclear cell recovery rate was 88%, and the filtration time was 4 minutes (flow rate: 25 cm$^3$/min).

[Example 6]

(1) Production of cell separation filter device

The same container as used in Example 3 was used. A filter material was produced from: a polyurethane porous body (sponge-structure) with an average pore diameter during packing of 12 $\mu$m acting as a first nucleated cell-capturing material; and a polyurethane porous body (sponge-structure) with an average pore diameter during packing

of 6 $\mu$m acting as a second recovery solution-rectifying material. Thereafter, from the inlet side of the above container, the above filter material was packed. It is to be noted that the packing thickness and packing porosity of the first layer were 2.2 mm and 60%, and that the packing thickness and packing porosity of the second layer were 0.7 mm and 60%. The value obtained by dividing the effective filtration area by the thickness of the nucleated cell-capturing material packed was 74. In addition, as in the case of Example 3, the polyurethane porous body was coated with a hydrophilic polymer.

(2) Cell separation system

The cell separation system shown in Figure 1 was used.

(3) Cell separation operation

The same cell separation operation as that in Example 3 was carried out. The amount of a cell solution recovered was 23 cm$^3$, and the time required for recovery was 3 seconds (flow rate: 380 cm$^3$/min).

(4) Results

In the present cell separation operation, the mononuclear cell recovery rate was 84%, and the filtration time was 6 minutes (flow rate: 16.7 cm$^3$/min).

[Comparative example 5]

(1) Production of cell separation filter device

The same container as used in Example 3 was used. With regard to a filter material, 1.67 g of a polyester non-woven fabric with an average fiber diameter of 1.7 $\mu$m acting as a nucleated cell-capturing material was packed to the above container. It is to be noted that the packing thickness and packing density of the above filter material were 2.9 mm and 0.25 g/cm$^3$. The value obtained by dividing the effective filtration area by the thickness of the nucleated cell-capturing material packed was 62.1 cm. In addition, as in the case of Example 3, the non-woven fabric was coated with a hydrophilic polymer.

(2) Cell separation system

The cell separation system shown in Figure 1 was used.

(3) Cell separation operation

The same cell separation operation as that in Example 3 was carried out. The amount of a cell solution recovered was 23 cm$^3$, and the time required for recovery was 3 seconds (flow rate: 380 cm$^3$/min).

(4) Results

In the present cell separation operation, the mononuclear cell recovery rate was 65%, and the filtration time was 7 minutes (flow rate: 14.3 cm$^3$/min).

[Comparative example 6]

(1) Production of cell separation filter device

The following container was prepared: a polycarbonate container consisting of an upper container and a lower container, which has an inside dimension after fabrication consisting of 22 mm long, 22 mm wide, and 12.4 mm high (effective filtration area: 5.3 cm$^2$; internal volume: 7 cm$^3$), and which has a liquid outlet and a liquid inlet on the longest diagonal line. A filter material was produced from: 1.50 g of a polyester non-woven fabric with an average fiber diameter of 1.7 $\mu$m acting as a first nucleated cell-capturing material; and 0.30 g of a polyester non-woven fabric with an average fiber diameter of 1.1 $\mu$m acting as a second recovery solution-rectifying material. Thereafter, from the inlet side of the above polycarbonate container, the above filter material was packed into the container. It is to be noted that the packing thickness and packing density of the first layer were 10 mm and 0.24 g/cm$^3$, and that the packing thickness and packing density of the second layer were 2.4 mm and 0.20 g/cm$^3$, respectively. The value obtained by dividing the effective filtration area by the thickness of the nucleated cell-capturing material filled was 5.3 cm. In addition, as in the case of Example 3, the non-woven fabric was coated with a hydrophilic polymer.

(2) Cell separation system

The cell separation system shown in Figure 1 was used.

(3) Cell separation operation

The same cell separation operation as that in Example 3 was carried out. However, after initiation of filtration, the flow rate sharply decreased, and the filtration was not completed after 1 hour or longer had passed.

[Comparative example 7]

(1) Production of cell separation filter device

The following container was prepared: a polycarbonate container consisting of an upper container and a lower container, which has an inside dimension after fabrication consisting of 74.2 mm long, 74.2 mm wide, and 3 mm high (effective filtration area: 55 cm$^2$; internal volume: 16 cm$^3$), and which has a liquid outlet and a liquid inlet on the longest diagonal line. A filter material was produced from: 3.61 g of a polyester non-woven fabric with an average fiber diameter of 1.7 $\mu$m acting as a first nucleated cell-capturing material; and 0.58 g of a polyester non-woven fabric with an average fiber diameter of 1.1 $\mu$m acting as a second recovery solution-rectifying material. Thereafter, from the outlet side of the above polycarbonate container, the above filter material was packed into the container. It is to be noted that the packing thickness and packing density of the first layer were 2.5 mm and 0.24 g/cm$^3$, and that the packing thickness and packing density of the second layer were 0.5 mm and 0.20 g/cm$^3$, respectively. The value obtained by dividing the effective filtration area by the thickness of the nucleated cell-capturing material packed was 220 cm. In addition, as in the case of Example 3, the non-woven fabric was coated with a hydrophilic polymer.

(2) Cell separation system

The cell separation system shown in Figure 1 was used.

(3) Cell separation operation

The same cell separation operation as that in Example 3 was carried out. The amount of a cell solution recovered was 21 cm$^3$, and the time required for recovery was 3 seconds (flow rate: 380 cm$^3$/min).

(4) Results

In the present cell separation operation, the mononuclear cell recovery rate was 55%, and the filtration time was 4 minutes (flow rate: 33.3 cm$^3$/min).

[Comparative example 8]

(1) Production of cell separation filter device

A single soft vinyl chloride sheet having a liquid-inlet port and a single soft vinyl chloride sheet having a liquid-outlet port were prepared. A filter material consisting of 1.37 g of a polyester non-woven fabric with an average fiber diameter of 1.7 $\mu$m acting as a first nucleated cell-capturing material and 0.19 g of a polyester non-woven fabric with an average fiber diameter of 1.1 $\mu$m acting as a second recovery solution-rectifying material was produced. Thereafter, the above filter material was sandwiched with the above two sheets from the above liquid-inlet port side. The surrounding portion thereof was welded with a high frequency, resulting in an effective filtration area of 18.5 cm$^2$, thereby producing a pouched filter device. This filter device was sandwiched with two acrylic plates such that the thickness of the first non-woven layer became 2.2 mm, and thus, the thickness was adjusted. The thickness of a non-woven fabric was adjusted by calibrating separately, using a filter device, which was produced in the same above manner, and it was adjusted using the space between two acrylic plates. In this case, the internal volume was 7 cm$^3$. The value obtained by dividing the effective filtration area by the thickness of the nucleated cell-capturing material filled was 74. In addition, the non-woven fabric used herein had been immersed in an ethanol solution that contained the same hydrophilic polymer as that in Example 1 before production of the above filter device, and it was then dried with a vacuum dryer at 60°C for 8 hours, before use.

(2) Cell separation system

The cell separation system shown in Figure 1 was used.

(3) Cell separation operation

Filtration was carried out in the same manner as in Example 3. During recovery, the space between the two acrylic plates for sandwiching the filter device was expanded, and the thickness of the nucleated cell-capturing material was adjusted to be 3.5 mm by the aforementioned calibration (internal volume: 11 cm$^3$; the packing density of the nucleated cell-capturing material: 0.17 g/cm$^3$). Thereafter, a syringe was filled with 23 cm$^3$ of a solution obtained by adding human serum albumin to a 10% dextran normal saline solution to a final concentration of 3%, and 18 cm$^3$ of air. The solution was manually supplied from the outlet for a cell-containing solution, and mononuclear cells were recovered from the inlet for a cell-containing solution. In this case, the value obtained by dividing the effective filtration area of the filter material by the thickness of the nucleated cell-capturing material filled was 51.4 cm. The amount of a cell solution recovered was 22 cm$^3$, and the time required for recovery was 5 seconds (flow rate: 276 cm$^3$/min).

(4) Results

In the present cell separation operation, the mononuclear cell recovery rate was 45%, and the filtration time was 6 minutes (flow rate: 16.7 cm$^3$/min).

[Comparative example 9]

(1) Production of cell separation filter device

The same filter device as that in Comparative example 8 was used.

(2) Cell separation system

The cell separation system shown in Figure 1 was used.

(3) Cell separation operation

The same operations as those in Comparative example 8 were carried out with the exception that 95 cm$^3$ of a solution obtained by adding human serum albumin to a 10% dextran normal saline solution to a final concentration of 3% and 18 cm$^3$ of air were used to prepare a recovery solution. The amount of a cell solution recovered was 100 cm$^3$, and the time required for recovery was 1 minute (flow rate: 100 cm$^3$/min).

(4) Results

In the present cell separation operation, the mononuclear cell recovery rate was 85%, and the filtration time was 6 minutes (flow rate: 16.7 cm$^3$/min).
The results of the above examples and comparative examples were summarized in Table 2.

[Table 2]

| | Effective filtration area / nucleated cell-capturing material (during filtration) | Effective filtration area / nucleated cell-capturing material (during recovery) | Presence or absence of recovery solution-rectifying material | Amount of recovered cell solution | Recovery rate of mononuclear cells | Filtration time |
|---|---|---|---|---|---|---|
| Example 3 | 74 cm | 74 cm | Present | 23 cm$^3$ | 85% | 6 min. |
| Example 4 | 16.7 cm | 16.7 cm | Present | 21 cm$^3$ | 75% | 10 min. |
| Example 5 | 84.1 cm | 84.1 cm | Present | 23 cm$^3$ | 88% | 4 min. |
| Example 6 | 74 cm | 74 cm | Present | 23 cm$^3$ | 70% | 6 min. |
| Comparative example 5 | 62.1 cm | 62.1 cm | Absent | 23 cm$^3$ | 65% | 7 min. |
| Comparative example 6 | 5.3 cm | 5.3 cm | Present | - | Non-recoverable | Non-filterable |
| Comparative example 7 | 220 cm | 220 cm | Present | 21 cm$^3$ | 55% | 4 min. |
| Comparative example 8 | 74 cm | 51.4 cm | Present | 22 cm$^3$ | 45% | 6 min. |
| Comparative example 9 | 74 cm | 51.4 cm | Present | 100 cm$^3$ | 85% | 6 min. |

Hereafter, methods of separating mononuclear cells from cord blood using a filter device, followed by centrifugation and cryopreservation, will be described in the following Example 7 and Comparative examples 10 and 11. In these cases, unnecessary cells are erythrocytes.

[Example 7]

(1) Production of cell separation filter device

The same filter device as that in Example 1 was used.

(2) Cell separation system

The cell separation system shown in Figure 1 was used.

(3) Storage bag

The storage bag shown in Figure 2 was used. The internal volume of the storage unit was 40 cm$^3$, and the internal volume of the cryopreservation unit was 5 cm$^3$.
As a material, an ethylene-vinyl acetate copolymer (EVA) was used.

(4) Cell concentration operation

Clamps 21, 22, and 23 had previously been closed. 100 cm$^3$ of CPD-added human cord blood stored in a 200-cm$^3$ cell-containing solution bag was connected with a blood conduit 11. The clamps 21 and 23 were opened, and filtration was initiated by head height gravity. The filtrated blood was stored in a drain bag 70. After confirming that all the blood in the cell-containing solution bag had been introduced into the cell separation filter device 40, the clamps 21 and 23 were closed. Subsequently, human serum albumin was added to a 10% dextran normal saline solution (Kobayashi Pharmaceutical Co., Ltd., "Dextran 40 Injection") to a final concentration of 3%. A syringe was then filled with 19 cm$^3$ of the obtained solution and 18 cm$^3$ of air. Thereafter, the clamp 22 was opened, the solution was manually supplied from a recovery solution-injecting port 60, and mononuclear cells were recovered in the storage bag having a cryopreservation unit 5. In this case, the amount of a cell solution recovered was 23 cm$^3$. The storage bag was separated from the cell separation system using a heat sealer. Thereafter, the thus separated storage bag was fixed such that a cryopreservation unit 120 can be located at the bottom of a centrifuge cup, and it was then centrifuged under centrifugal conditions of 1,000 G x 15 minutes. The supernatant filled up to a narrowed area (meltdown separation unit) 110 was eliminated. While discharging the air out of the cryopreservation unit 120, it was separated by melting down using a heat sealer at the meltdown separation unit 110, so as to separate it from the storage unit 100. Thereafter, while holding the cryopreservation unit 120 with fingers, the precipitate was dispersed. While cooling the surface of the cryopreservation unit 120 with a cooler, 1 cm$^3$ of 50% dimethyl sulfoxide was added thereto from a cryoprotective agent-introducing unit 130 using a syringe pump at a flow rate of 0.3 cm$^3$/min. The final volume was 5 cm$^3$. Thereafter, the obtained mixture was stood at rest at -80°C for 2 hours, and it was preserved in a frozen state in a gas phase of liquid nitrogen.

(5) Analysis

The sample was taken out from a nucleated cell-discharging port 140 before cryopreservation, and it was then mixed with the original cord blood. Thereafter, the number of cells was counted using a sequential multi channel automatic blood cell analyzer (SF3000, Sysmex), so as to calculate the recovery rate.

(6) Results

In the present cell concentration operation, the mononuclear cell recovery rate was 85%. After thawing the frozen product, the breakage of the cryopreservation unit 120 was not observed.

[Comparative example 10]

Mononuclear cells separated by the filter method were transferred into a conical tube, followed by centrifugal concentration. The obtained mononuclear cell concentrated solution was further transferred into a storage bag, and it was preserved in a frozen state.

(1) Production of cell separation filter device

The same filter device as that in Example 7 was used.

(2) Cell separation system

A common blood bag, which does not have the narrowed area 110 and cryopreservation unit 120 in the present invention, was used instead of the storage bag in the cell separation system shown in Figure 1.

(3) Storage bag

The same storage bag as that used in Example 7 was used.

(4) Cell separation operation

As with Example 7, the cord blood was treated by the cell separation system shown in Figure 1, and the nucleated cells were recovered in a common blood bag, which does not have the narrowed area 110 and cryopreservation unit 120 in the present invention. 23 cm$^3$ of the recovered nucleated cell solution was transferred into two 14cm$^3$ conical tubes, and it was then centrifuged under centrifugal conditions of 1,000 G x 15 minutes. The supernatant was removed, resulting in a volume of 4 cm$^3$, and the precipitate was suspended again. Thereafter, while cooling with a cooler, 1 cm$^3$ of 50% dimethyl sulfoxide was added thereto at a flow rate of 0.3 cm$^3$/min. Thereafter, the obtained cell concentrate was transferred into the storage bag shown in Figure 2, and it was then preserved in a frozen state in the same manner as in Example 7.

(5) Results

In the present cell concentration operation, the mononuclear cell recovery rate was 50%. After thawing the frozen product, the breakage of the cryopreservation unit 120 was not observed.

[Comparative example 11]

In Example 7, cryopreservation was carried out without an operation to discharge the air out of the cryopreservation unit 120.

(1) Production of cell separation filter device

The same filter device as that in Example 7 was used.

(2) Cell separation system

The cell separation system shown in Figure 1 was used.

(3) Storage bag

The same storage bag as that used in Example 7 was used.

(4) Cell separation operation

The operations were carried out in the same manner as in Example 7 with the exception that 2 cm$^3$ of air was filled in the cryopreservation unit 120 before cryopreservation.

(5) Results

In the present cell concentration operation, the mononuclear cell recovery rate was 85%. After thawing the frozen product, the breakage of the cryopreservation unit 5 was observed.

[INDUSTRIAL APPLICABILITY]

The method for preparing a cell concentrate according to the present invention is used as a means for pretreatment for cryopreservation of cells. Since this method enables simple and efficient separation of nucleated cells such as mononuclear cells from unnecessary cells such as erythrocytes contained in a cell-containing solution such as bone marrow, cord blood, or peripheral blood, thereby significantly reducing the volume of a cryopreservation solution that contains the nucleated cells. This is a method that is useful for cryopreservation of nucleated cells, and thus it can be used as an effective means for reducing a preservation cost. Moreover, the method for preserving cells in a frozen state according to the present invention can be used to separate nucleated cells such as mononuclear cells from unnecessary cells such as erythrocytes contained in a cell-containing solution such as bone marrow, cord blood, or peripheral blood, and to preserve such cells in a frozen state. Thus, this method can be used as an effective means for reducing the loss of nucleated cells, a risk of bacteria contamination, or a risk of the breakage of a freezing container.

[BRIEF DESCRIPTION OF THE DRAWINGS]

Figure 1 is a schematic diagram showing an example of a system for carrying out the method for preparing a cell concentrate according to the present invention.
Figure 2 is a schematic diagram showing an example of the shape of the storage bag of the present invention.
Figure 3 is a schematic diagram showing another example of the shape of the storage bag of the present invention.
Figure 4 is a schematic diagram showing a further example of the shape of the storage bag of the present invention.

**Claims**

**1.** A method for preparing a cell concentrate, which comprises:

introducing a cell-containing solution that contains (i) nucleated cells and (ii) erythrocytes as unnecessary cells into a filter device comprising a filter material for substantially capturing the nucleated cells and for substantially giving passage to the unnecessary cells, so as to capture the nucleated cells by the above-described filter material and to discharge the unnecessary cells from the above device; and introducing a recovery solution into the above-described filter device, so as to recover the nucleated cells captured by the above-described filter material,

wherein the above-described method is **characterized in that** a blood bag that contains a cell-containing solution that contains nucleated cells and unnecessary cells is connected to a previously closed blood conduit of the filter device, the cell-containing solution that contains nucleated cells and unnecessary cells is separated into a layer that is rich in nucleated cells and a layer that is rich in unnecessary cells, then the blood conduit is opened; the layer rich in unnecessary cells is first introduced into the above-described filter device, and the layer rich in nucleated cells is then introduced therein, so as to discharge the unnecessary cells remaining in the above-described filter device while capturing the nucleated cells by the above-described filter material, and a recovery solution is then introduced into the above-described filter device, so as to recover the nucleated cells captured by the above-described filter material.

2. The method for preparing a cell concentrate according to claim 1, wherein unnecessary cells are precipitated by gravity or centrifugal force, so as to separate a cell-containing solution that contains at least nucleated cells and unnecessary cells into a layer that is rich in nucleated cells and a layer that is rich in unnecessary cells.

3. The method for preparing a cell concentrate according to claim 1, wherein unnecessary cells are agglutinated and are then precipitated by gravity or centrifugal force, so as to separate a cell-containing solution that contains at least nucleated cells and unnecessary cells into a layer that is rich in nucleated cells and a layer that is rich in unnecessary cells.

4. The method for preparing a cell concentrate according to any one of claims 1 to 3, wherein the above-described nucleated cells are hematopoietic stem cells.

5. The method for preparing a cell concentrate according to claim 3, wherein unnecessary cells are agglutinated by adding hydroxyethyl starch to a cell-containing solution that contains nucleated cells and unnecessary cells.

6. The method for preparing a cell concentrate according to any one of claims 1 to 5,wherein the above-described layer that is rich in nucleated cells consists of a nucleated cell-concentrated layer and a nucleated cell-diluted layer, and wherein the nucleated cell-diluted layer and the nucleated cell-concentrated layer are introduced into the filter device in this order.

7. The method for preparing a cell concentrate according to any one of claims 1 to 4, wherein the above-described layer that is rich in nucleated cells consists of a nucleated cell-concentrated layer and a nucleated cell-diluted layer, and wherein the nucleated cell-concentrated layer and the nucleated cell-diluted layer are introduced into the filter device in this order.

8. The method for preparing a cell concentrate according to claim 7, wherein a part of or all of the above-described nucleated cell-diluted layer is used as at least a portion of a recovery solution.

9. The method for preparing a cell concentrate according to any one of claims 1 to 8, wherein a recovery solution is introduced into the above-described filter device to recover nucleated cells captured by the above-described filter material, and the recovered nucleated cell-containing solution is further centrifuged to eliminate the supernatant thereof, so as to concentrate the nucleated cells.

10. The method for preparing a cell concentrate according to any one of claims 1 to 9, which is **characterized in that** the above-described filter material is configured such that a container having at least an inlet and an outlet for a cell-containing solution is packed with a nucleated cell-capturing material and a recovery solution-rectifying material, which consist of porous bodies, in this order in a direction from the inlet side to the outlet side for a cell-containing solution, and **in that** the value obtained by dividing the effective filtration area of the above-described filter material by the thickness of the nucleated cell-capturing material packed is between 15 and 120 cm.

**Patentansprüche**

1. Verfahren zur Herstellung eines Zellkonzentrats, umfassend: das Einführen einer zellhaltigen Lösung, die (i) kernhaltige Zellen und (ii) Erythrocyten als unnötige Zellen enthält, in eine Filtervorrichtung, die ein Filtermaterial umfasst, das die kernhaitigen Zellen im Wesentlichen abfängt und die unnötigen Zellen im Wesentlichen durchlässt, so dass die kernhaltigen Zellen durch das oben beschriebene Filtermaterial abgefangen werden und die unnötigen Zellen aus der obigen Vorrichtung entfernt werden, und das Einführen einer Rückgewinnungslösung in die oben beschriebene Filtervorrichtung, so dass die durch das oben beschriebene Filtermaterial abgefangenen kernhaltigen Zellen zurückgewonnen werden;

wobei das oben beschriebene Verfahren **dadurch gekennzeichnet ist, dass** ein Blutbeutel, der eine zellhaltige Lösung enthält, die kernhaltige Zellen und unnötige Zellen enthält, an eine zuvor geschlossene Blutleitung der Filtervorrichtung angeschlossen wird, die zellhaltige Lösung, die kernhaltige Zellen und unnötige Zellen enthält, in eine Schicht, die reich an kernhaltigen Zellen ist, und eine Schicht, die reich an unnötigen Zellen ist, aufgetrennt wird, die Blutleitung dann geöffnet wird; die Schicht, die reich an unnötigen Zellen ist, zuerst in die oben beschriebene Filtervorrichtung eingeführt wird, und die Schicht, die reich an kernhaltigen Zellen ist, danach dort eingeführt wird, so dass die in der oben beschriebenen Filtervorrichtung verbliebenen unnötigen Zellen entfernt werden, während die kernhaltigen Zellen durch das oben beschriebene Filtermaterial abgefangen werden, und dann eine Rückgewinnungslösung in die oben beschriebene Filtervorrichtung eingeführt wird, so dass die durch das oben beschriebene Filtermaterial abgefangenen kernhaltigen Zellen zurückgewonnen werden.

2. Verfahren zur Herstellung eines Zellkonzentrats gemäß Anspruch 1, wobei unnötige Zellen durch die Schwerkraft oder eine Zentrifugalkraft sedimentiert werden, so dass eine zellhaltige Lösung, die wenigstens kernhaltige Zellen und unnötige Zellen enthält, in eine Schicht, die reich an kernhaltigen Zellen ist, und eine Schicht, die reich an unnötigen Zellen ist, aufgetrennt wird.

3. Verfahren zur Herstellung eines Zellkonzentrats gemäß Anspruch 1, wobei unnötige Zellen agglutiniert und dann durch die Schwerkraft oder eine Zentrifugalkraft sedimentiert werden, so dass eine zellhaltige Lösung, die wenigstens kernhaltige Zellen und unnötige Zellen enthält, in eine Schicht, die reich an kernhaltigen Zellen ist, und eine Schicht, die reich an unnötigen Zellen ist, aufgetrennt wird.

4. Verfahren zur Herstellung eines Zellkonzentrats gemäß einem der Ansprüche 1 bis 3, wobei die oben beschriebenen kernhaltigen Zellen hämatopoetische Stammzellen sind.

5. Verfahren zur Herstellung eines Zellkonzentrats gemäß Anspruch 3, wobei unnötige Zellen **dadurch** agglutiniert werden, dass man Hydroxyethylstärke zu einer zellhaltigen Lösung gibt, die kernhaltige Zellen und unnötige Zellen enthält.

6. Verfahren zur Herstellung eines Zellkonzentrats gemäß einem der Ansprüche 1 bis 5, wobei die oben beschriebene Schicht, die reich an kernhaltigen Zellen ist, aus einer an kernhaltigen Zellen angereicherten Schicht und einer bezüglich kernhaltiger Zellen verdünnten Schicht besteht und wobei die bezüglich kernhaltiger Zellen verdünnte Schicht und die an kernhaltigen Zellen angereicherte Schicht in dieser Reihenfolge in die Filtervorrichtung eingeführt werden.

7. Verfahren zur Herstellung eines Zellkonzentrats gemäß einem der Ansprüche 1 bis 4, wobei die oben beschriebene Schicht, die reich an kernhaltigen Zellen ist, aus einer an kernhaltigen Zellen angereicherten Schicht und einer bezüglich kernhaltiger Zellen verdünnten Schicht besteht und wobei die an kernhaltigen Zellen angereicherte Schicht und die bezüglich kernhaltiger Zellen verdünnte Schicht in dieser Reihenfolge in die-Filtervorrichtung eingeführt werden.

8. Verfahren zur Herstellung eines Zellkonzentrats gemäß Anspruch 7, wobei ein Teil oder die gesamte oben beschriebene bezüglich kernhaltiger Zellen verdünnte Schicht als wenigstens ein Teil der Rückgewinnungslösung verwendet wird.

9. Verfahren zur Herstellung eines Zellkonzentrats gemäß einem der Ansprüche 1 bis 8, wobei eine Rückgewinnungslösung in die oben beschriebene Filtervorrichtung eingeführt wird, so dass durch das oben beschriebene Filtermaterial abgefangene kernhaltige Zellen zurückgewonnen werden, und die Lösung, die die zurückgewonnenen kernhaltigen Zellen enthält, weiterhin zentrifugiert wird, um den Überstand zu entfernen und **dadurch** die kernhaltigen Zellen anzureichern.

10. Verfahren zur Herstellung eines Zellkonzentrats gemäß einem der Ansprüche 1 bis 9, das **dadurch gekennzeichnet ist, dass** das oben beschriebene Filtermaterial so konfiguriert ist, dass ein Behälter, der wenigstens einen Einlass und einen Auslass für eine zellhaltige Lösung aufweist, mit einem kernhaltige Zellen abfangenden Material und einem die Rückgewinnungslösung rektifizierenden Material, das aus porösen Körpern besteht, in dieser Reihenfolge in einer Richtung von der Einlassseite zur Auslassseite für eine zellhaltige Lösung gefüllt wird, und dass der Wert, den man durch Dividieren der effektiven Filterfläche des oben beschriebenen Filtermaterials durch die Dicke des eingefüllten, die kernhaltigen Zellen abfangenden Materials erhält, zwischen 15 und 120 cm liegt.

**Revendications**

1. Procédé de préparation d'un concentré cellulaire, comprenant : l'introduction d'une solution contenant des cellules, qui contient (i) des cellules nucléées et (ii) des érythrocytes en tant que cellules inutiles, dans un dispositif de filtration comprenant un matériau de filtration pour essentiellement capturer les cellules nucléées et pour essentiellement laisser passer les cellules inutiles, afin de capturer les cellules nucléées avec le matériau de filtration décrit ci-dessus et pour décharger les cellules inutiles du dispositif ci-dessus ; et l'introduction d'une solution de récupération dans le dispositif de filtration décrit ci-dessus, pour récupérer les cellules nucléées capturées par le matériau de filtration décrit ci-dessus, dans lequel le procédé décrit ci-dessus est **caractérisé en ce qu'**une poche de sang qui contient une solution contenant des cellules qui contient des cellules nucléées et des cellules inutiles est raccordée à un conduit pour sang préalablement fermé du dispositif de filtration, la solution contenant des cellules qui contient des cellules nucléées et des cellules inutiles est séparée en une phase riche en cellules nucléées et une phase riche en cellules inutiles, puis le conduit pour sang est ouvert ; la phase riche en cellules inutiles est introduite la première dans le dispositif de filtration décrit ci-dessus et la phase riche en cellules nucléées y est ensuite introduite, pour décharger les cellules inutiles restant dans le dispositif de filtration décrit ci-dessus tout en capturant les cellules nucléées avec le matériau de filtration décrit ci-dessus, et une solution de récupération est ensuite introduite dans le dispositif de filtration décrit ci-dessus pour récupérer les cellules nucléées capturées par le matériau de filtration décrit ci-dessus.

2. Procédé de préparation d'un concentré cellulaire selon la revendication 1, dans lequel les cellules inutiles sont précipitées sous l'action de la gravité ou d'une force centrifuge, pour séparer une solution contenant des cellules qui contient au moins des cellules nucléées et des cellules inutiles en une phase riche en cellules nucléées et une phase riche en cellules inutiles.

3. Procédé de préparation d'un concentré cellulaire selon la revendication 1, dans lequel les cellules inutiles sont agglutinées et sont ensuite précipitées sous l'action de la gravité ou d'une force centrifuge, pour séparer une solution contenant des cellules qui contient au moins des cellules nucléées et des cellules inutiles en une phase riche en cellules nucléées et une phase riche en cellules inutiles.

4. Procédé de préparation d'un concentré cellulaire selon l'une quelconque des revendications 1 à 3, dans lequel les cellules nucléées décrites ci-dessus sont des cellules souches hématopoïétiques.

5. Procédé de préparation d'un concentré cellulaire selon la revendication 3, dans lequel les cellules inutiles sont agglutinées par l'ajout d'un amidon hydroxyéthylé à une solution contenant des cellules qui contient des cellules nucléées et des cellules inutiles.

6. Procédé de préparation d'un concentré cellulaire selon l'une quelconque des revendications 1 à 5, dans lequel la phase décrite ci-dessus qui est riche en cellules nucléées est constituée d'une phase concentrée de cellules nucléées et d'une phase diluée de cellules nucléées, et dans lequel la phase diluée de cellules nucléées et la phase concentrée de cellules nucléées sont introduites dans le dispositif de filtration dans cet ordre.

7. Procédé de préparation d'un concentré cellulaire selon l'une quelconque des revendications 1 à 4, dans lequel la phase décrite ci-dessus qui est riche en cellules nucléées est constituée d'une phase concentrée de cellules nucléées et d'une phase diluée de cellules nucléées, et dans lequel la phase concentrée de cellules nucléées et la phase diluées de cellules nucléées sont introduites dans le dispositif de filtration dans cet ordre.

8. Procédé de préparation d'un concentré cellulaire selon la revendication 7, dans lequel une partie ou la totalité de la phase diluée de cellules nucléées décrite ci-dessus est utilisée pour former au moins une partie d'une solution de récupération.

9. Procédé de préparation d'un concentré cellulaire selon l'une quelconque des revendications 1 à 8, dans lequel une solution de récupération est introduite dans le dispositif de filtration décrit ci-dessus pour récupérer les cellules nucléées capturées par le matériel de filtration décrit ci-dessus, et la solution contenant les cellules nucléées récupérées est encore centrifugée pour éliminer son surnageant, afin de concentrer les cellules nucléées.

10. Procédé de préparation d'un concentré cellulaire selon l'une quelconque des revendications 1 à 9, qui est **caractérisé en ce que** le matériau de filtration décrit ci-dessus est conçu de telle sorte qu'un récipient possédant au moins un orifice d'entrée et un orifice de sortie pour une solution contenant des cellules est rempli avec un matériau de capture de cellules nucléées et un matériau de rectification de solution de récupération, qui sont constitués de corps poreux, dans cet ordre dans une direction allant du côté de l'orifice d'entrée vers le côté de l'orifice de sortie pour une solution contenant des cellules, et **en ce que** la valeur obtenue en divisant la surface de filtration efficace du matériau de filtration décrit ci-dessus par l'épaisseur du matériau de capture de cellules nucléées introduit est située entre 15 cm et 120 cm.

Fig. 1

Fig. 2

Fig. 3

Fig. 4

**EP 1 683 857 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 10201470 A
- JP 10137557 A
- JP 11206875 A
- JP 11056351 A
- WO 9832840 A
- WO 02087660 A
- JP 57083284 A
- JP 6051060 A
- JP 2013588 A

### Non-patent literature cited in the description

- *Proc. Natl. Acad. Sci. U.S.A.,* 1995, vol. 92, 10119-10122
- *Indian J. Med. Res.,* 1997, vol. 106, 16-19
- *Bone Marrow Transplantation,* 1999, vol. 23, 505-509